# EUROPEAN PATENT APPLICATION

(11) **EP 2 179 980 A1**
(43) Date of publication of application: **28.04.2010**
(21) Application number: 08018542.4
(22) Date of filing: 23.10.2008
(51) Int. Cl.: C07C 5/10, C07C 67/303, C07C 13/18, C07C 69/74

(54) **Process for the manufacture of saturated mono- or polycyclic compounds**

(71) Applicant: Cognis IP Management GmbH, 40589 Düsseldorf (DE); National & Kapodistrian University of Athens, 10679 Athens (GR)
(72) Inventor: Vangelis, Constantinos, 48200 Filippiada, Prevea (GR); Sotiriou, Sotiris, 16231 Vironas, Athen (GR); Yokaris, Alexandros, 15343 Agia Paraskevi, Athen (GR); Bouriazos, Achilleas, 18542 Palaia Kokkínía, Peíreas (GR); Papadogianakis, Georgios, 13121 Ilion, Athen (GR)
(74) Representative: Reinhardt, Jürgen

(57) **Abstract**

Suggested is a process for the manufacture of saturated mono- or polycyclic compounds via hydrogenation of the respective unsaturated mono- or polycyclic compounds, which is **characterised in that** the reaction is conducted in an aqueous/organic two phase system in the presence of a water soluble catalyst consisting of a Group VIII metal and a hydrophilic ligand.

## Description

The present invention relates to the area of catalytic hydrogenation of aromatics to produce hydrogenated cyclic compounds. More particularly this invention relates to an improved biphasic catalytic process for the selective hydrogenation of aromatic compounds employing highly active and stable water soluble transition metal complexes, the hydrogenated cyclic compounds thus obtained, and their use in the area of the manufacture of nylon 6 and nylon 6.6 polyamides, of the removal of benzene of gasoline and of other aromatics contained in diesel fuel, of the manufacture of improved stability of specialty polyamide oligomers (Versamids) coatings, epoxy hardeners and lubricants based on dimer fatty acid alkyl esters or alcohols, and of the inhibition of oxidative yellowing of paper made from mechanical pulps.

### Background of the invention

The hydrogenation of benzene to cyclohexane is one of the most important industrially practiced aromatic compounds hydrogenation reactions. Millions of tonnes of benzene are hydrogenated to cyclohexane per year and about 90% of world's production of cyclohexane is used for manufacturing nylon 6 and nylon 6.6 which are about 90% of all polyamides. Cyclohexane was first obtained by the direct fractional distillation of suitable crude petroleum refinery streams with the purity being only 85%. Further purifications improved the product quality to 98% as a result of the simultaneous isomerization reaction of methylcyclopentane to cyclohexane as operated by Atlantic Richfield, Shell and Humble Oil. Nowadays, almost the whole world's production capacity of cyclohexane is manufactured by the route of the Hydrogenation of benzene. However, despite its apparent simplicity the hydrogenation reaction of benzene has evolved through many variations and has given rise to many different processes. The successful industrial hydrogenation of benzene suitable for petrochemical cyclohexane production requires the resolution of three major critical problems: (i) the reaction is strongly exothermic with a Δ*H*⁰_{f (473 K)} of - 214.2 kJ. mol⁻¹, (ii) the cyclohexane must be pure, and (iii) the low stability of *e*.*g*. nickel catalysts which require extremely pure benzene feedstocks with less than 1 ppm sulphur in order that the catalysts remain effective in the liquid-phase under mild conditions. The strongly exothermic hydrogenation reaction requires careful control of temperature, pressure and residence time in order to achieve quantitative conversion of benzene at very high cyclohexane selectivity. Thus, significant formation of methylcyclopentane, which is favoured at higher temperatures, has to be suppressed. Typical cyclohexane specifications require a content of methylcyclopentane of less than 200 ppm and of unreacted benzene content of less than 100 ppm. Conventionally, the hydrogenation of benzene leads to the production of methylcyclopentane, methylpentane, n-paraffins (n-hexane n-pentane, etc.) and methane as predominant byproducts obtained by isomerization and hydrocracking side reactions. The formation of these undesired by-products is likewise favoured at relatively high reaction temperatures and like methylcyclopentane they can be separated from the cyclohexane product only by complicated separation methods such as rectification, extraction and employing molecular sieves. The interest in reducing the amount of byproducts comes mainly from the end users of nylon 6 and nylon 6.6 who require very low levels of byproducts in the polyamides in order to improve their properties. One method of decreasing the level of by-products in ε-caprolactam and adipic acid and subsequently in the nylon 6 and nylon 6.6 end-products is the reduction of the amount of by-products in cyclohexane.

There are numerous industrial processes for the hydrogenation of benzene to cyclohexane which are carried out in the liquid or gas phase. In 1995, the world production capacity of cyclohexane was 5.1 x 10⁶ t/a. Among the well known liquid phase benzene hydrogenation processes are the Mitsubishi, Sinclair and the Institut Francais du Petrole (IFP) process. The IFP process uses a Raney nickel catalyst in a bubble column reactor at temperatures between 200 and 230°C under a hydrogen pressure which must be sufficient to maintain a liquid phase i. e. 50 bar. Residual benzene, which is about 5%, is quantitatively converted in a followed fixed bed hydrogenation reactor in the gas phase. In 1991, 23 industrial facilities were operated worldwide according to the IFP benzene hydrogenation process in the liquid phase with a production capacity of 1.8 x 10⁶ t/a. The main drawbacks with this process are due to the fact that the Raney nickel catalyst posses a low stability towards sulphur compounds unavoidable in benzene feedstock and that the catalyst is pyrophoric to a greater or less extent. Benzene feedstock requires deep desulphurization to sulphur content of < 1 ppm which considerably increases the cost of the whole process. Well known gas phase benzene hydrogenation processes were developed by several companies like UOP (Hydrar process), DSM, Toray, Arco and Houdry under forcing conditions (400 - 600°C, P_{H2} = 30 bar) and short residence times in order to avoid side reactions.

A general trend is to reduce the quantities of benzene in gasoline and of other aromatic compounds in diesel fuel because of their known toxic properties. Thus, their possibilities of polluting the air must be limited as far as possible in particular by practically excluding aromatics from automobile fuels. The benzene content in gasoline depends largely on the reformate component in that gasoline. The reformate results from catalytic treatment of naphtha intended to produce aromatics comprising mainly 6 to 9 C atoms per molecule which exhibit very high octane number giving the gasoline antiknock properties. The reformate benzene could be hydrogenated to cyclohexane. However, this hydrogenation reaction can not be performed in a mixture of hydrocarbons containing toluene and xylenes. This mixture must be first fractionated to isolate the cut of benzene which could then be hydrogenated.

High aromatic content in diesel fuel has been recognized that they contribute to higher emissions and their presence lower the quality of the fuel. Indeed, aromatics are known to reduce the cetane number of diesel fuels and generate more smoke and soot particles during combustion. More and more environmental regulations governing diesel fuel specifications are set up to limit the aromatic content to low levels and therefore new catalysts and processes for aromatics hydrogenation in middle distillates are receiving increasing attention. The hydrogenation of aromatic compounds is more difficult to be achieved than hydrodesulphurization and hydrodenitrogenation over conventional hydrotreating catalysts under conditions that are normally used for lowering sulphur and nitrogen levels. The hydrogenation of polyaromatics to monoaromatics occurs even at low severity but the monoaromatics stay nearly unchanged. The hydrogenation of monoaromatics is the key step for the production of low aromatic diesel fuel. Numerous processes have been developed for deep aromatic hydrogenation in diesel fuel e.g. by IFP, Shell, Criterion, Criterion jointly with Lummus Crest Inc. (SynSat process), and Haldor Topsøe.

Dimer fatty acids and their derivatives are prone to autoxidation because they contain olefinic and aromatic C=C units and hydrogenation reactions increase the oxidation and thermal stabilities of such compounds which are primarily used in hot glues, printing colours, polyamide oligomers (Versamids) coatings and epoxy hardeners. Dimer fatty acids are manufactured by dimerizations reactions of C₁₈ fatty acids with one or more double bonds which probably react according to Diels-Alder reactions at temperatures 210-250 °C in the presence of montmorillonit catalytic systems [Chemie in unserer Zeit, 26, p27f (1992); Angew. Chem. 100, p41f (1988)]. Hydrogenation reactions of C=C units in acyclic and cyclic olefinic and aromatic moieties of dimer fatty acids and their derivatives result in a solid phase containing straight chain fatty acids and a liquid phase containing technical isostearic acids and are ideal raw materials for e.g. lubricants, cosmetics and plastic additives possessing excellent thermal and oxidation resistance. Conventional commercial heterogeneous catalysts such as Pd/C are capable to hydrogenate effectively C=C olefinic units of dimer fatty acids and their derivatives.

However, such conventional heterogeneous catalytic systems show lower activity in the hydrogenation of aromatic compounds contained in dimer fatty acids and their derivatives.

Mechanical pulping technology shows many positives features, *inter alia,* the efficient utilization of wood or non-wood materials, much lower operating and capital costs, compared with chemical pulping technology and also the potential to produce pulp in a zero liquid effluent process. However, the use of mechanical pulps as constituents in some paper products is severely restricted by the well known tendency of mechanical pulps to undergo colour reversion, i.e. yellowing, upon exposure to daylight and during storage at ambient conditions probably due to photooxidation processes of lignin. A number of approaches have been proposed for the inhibition of oxidative yellowing of mechanical pulps among them hydrogenation reactions of aromatic rings of lignin model compounds catalyzed by rhodium nanoparticles in monophasic aqueous-ethanol media **[**J. Pulp Paper Sci., 23, p J200f (1997**)].**

There are numerous catalytic hydrogenations of benzene in aqueous/organic two-phase systems described in the literature. All these aqueous/organic biphasic reactions are catalyzed either by nanoparticles and clusters when the catalysts stay in the aqueous phase or by homogeneous complexes which are soluble in the upper organic phase. Furthermore, many transition metal catalytic complexes originally assigned as homogeneous in the hydrogenation of aromatics have subsequently been shown to be heterogeneous catalytic metal-particles **[**Dalton Trans., p2964f (2003**);** J. Mol. Catal. A: Chem., 198, p 317f (2003**)].**

There are only four reports on the industrially important biphasic hydrogenation of benzene using water soluble transition metal complexes modified with sulphonated phosphines. Two papers are devoted to the ruthenium/ trisulphonated triphenylphosphine (TPPTS)-, one on the ruthenium/disulphonated triphenylphosphine (TPPDS)- and another one on the tungsten/monosulphonated triphenylphosphine (TPPMS)-catalyzed biphasic hydrogenation of benzene. In these biphasic reactions, however, Ru/TPPTS exhibited very low catalytic activity namely 0.6 TOF's per hour. Similar, the Ru/TPPDS catalyst exhibited low activity namely 60 TOF's per hour and W/TPPMS catalyst exhibited also very low catalytic activity i.e. 0.4 TOF's per hour.

It has therefore the object of the present invention to overcome the disadvantages known from the state of the art and to provide a cost-effective process for hydrogenation of aromatic compounds which involves a highly active, selective and stable homogenous catalytic system based on transition metals modified with water soluble ligands which stays in water and allows to perform the reaction in an aqueous/organic two-phase system which provides the means for the quantitative recovery of the water soluble catalyst by a simple phase separation and perform the reaction under mild conditions.

### Detailed description of the invention

The present invention refers to a process for the manufacture of saturated mono- or polycyclic compounds via hydrogenation of the respective unsaturated mono- or polycyclic compounds, which is **characterised in that** the reaction is conducted in an aqueous/organic two phase system in the presence of a water soluble catalyst consisting of a Group VIII metal and a hydrophilic ligand.

Surprisingly it has been found that homogenous catalysts consisting of Group VIII metals and hydrophilic ligands allow the selective hydrogenation of monocyclic or polycyclic aromatic compounds in an aqueous/organic two phase system with extremely high turnover frequency (TOF). The hydrogenation provides hydrogenated cyclic compounds which reaction could be used in the area of the manufacture of nylon 6 and nylon 6.6 polyamides, of the removal of benzene of gasoline and of other aromatics contained in diesel fuel, of the manufacture of improved stability of specialty polyamide oligomers (Versamids) coatings, epoxy hardeners, lubricants and cosmetics based on dimer fatty acid alkyl esters or alcohols, and of the inhibition of oxidative yellowing of paper made from mechanical pulps negating the need for environmentally damaging bleaches. Beside this another advantage of the catalysts is seen in that they can be recycled from the reaction mixture by simple phase separation and transferred back into the hydrogenation without loss of activity. Moreover, dynamic light scattering studies on a five times recycled aqueous Rh/TPPTS catalyst solution of the biphasic benzene hydrogenation (example 47) and the pH effect of the same reaction (examples 42-44) suggested that the Rh/TPPTS catalyst is of a homogeneous nature in aqueous media without formation of any heterogeneous rhodium catalytic nanoparticles.

### Mono- or polycyclic compounds

The selection of the cyclic unsaturated, usually aromatic mono- or polycyclic starting material is not critical, since the process according to the present invention is suitable to convert all of these unsaturated starting materials into their saturated homologues. Typically, suitable starting materials follow the following formulas 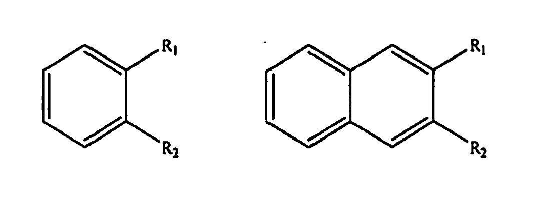 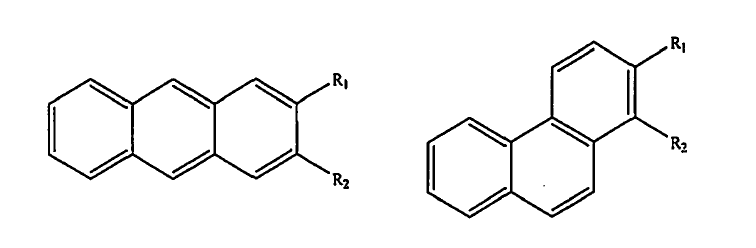 in which R¹ and R² independently from each other represent hydrogen or an alkyl, ester, alkoxy, aldehyde, keto, acid, amide, nitril, amino, nitro, halide, and/or hydroxy group. Also included are those geometric isomers of the cyclic compounds described above, that means those isomers which do not stand in 1,2 position, but in each other position to the first substituent, since the distribution of the substituents at the ring system does not have an influence on the hydrogenation process. Also encompassed are those cyclic compounds which do not show an aromatic behaviour, since they only comprise one or two double bonds. Even cyclic compounds like cyclopentadien or dicyclopentadien can be converted in the respective saturated compounds by means of the present process. The preferred cyclic starting materials, however, are benzene, toluene and dimer fatty acids or their esters, which are for example obtainable from the dimerisation of oleic acid.

### Catalysts

Currently there is growing interest in catalysis in aqueous media using water soluble transition metal complexes because of its broad range of potential applications. Water soluble catalysts in an aqueous/organic two phase system combine the advantages of homogeneous and heterogeneous catalysis:
(i) high activities and selectivities under mild reaction conditions by tailoring of the coordination sphere of the transition metal;
(ii) easy and quantitative recovery of the catalyst in active form from organic reaction products by simple phase separation.

Interest in aqueous systems has been further stimulated by increasing environmental constrains. Thus, aqueous/organic systems are environmentally attractive for the following reasons:
(i) numerous steps for the catalyst recovery in classical homogeneous catalytic processes are rendered superfluous and process engineering is enormously simplified, resulting in substantial energy savings and lower emissions;
(ii) they obviate the need for toxic organic solvents and water is a non-toxic, non-inflammable, safe, inexpensive, widely available and environmentally friendly, "green", solvent.

Presently, there are four industrial processes employing water soluble transition metal complexes as catalysts:
(i) the Ruhrchemie/Rhône-Poulenc process for the hydroformylation of lower olefins such as propene and butenes catalysed by Rh/TPPTS complexes in aqueous/organic two phase systems with a capacity of 800000 t/a;
(ii) the Rhône-Poulenc process for the synthesis of vitamin E and A intermediates using rhodium catalysts modified with TPPTS in a two phase system;
(iii) the Kuraray process for the synthesis of 1.9-nonanediol and n-octanol using palladium and rhodium catalysts modified with TPPMS in a two phase system; and finally
(iv) the Union Carbide pilot plant process for the hydroformylation of higher olefins in a one-phase system with biphasic separation of the Rh/TPPMS catalyst.

In conjunction with the catalyst, it has been proven advantageous if the water soluble metal complexes are compounds whose central atom is a Group VIII metal, in particular rhodium, ruthenium, palladium, cobalt, platinum, nickel, iridium, iron, and preferably rhodium, ruthenium and platinum.

The water soluble metal complex preferably contains hydrophilic ligands, especially hydrophilic phosphines, amines and oxygen containing ligands, preferably sulphonated mono-phosphines and sulphonated chelating phosphines. Suitable hydrophilic ligands (numbered 1-224) are given in the following:

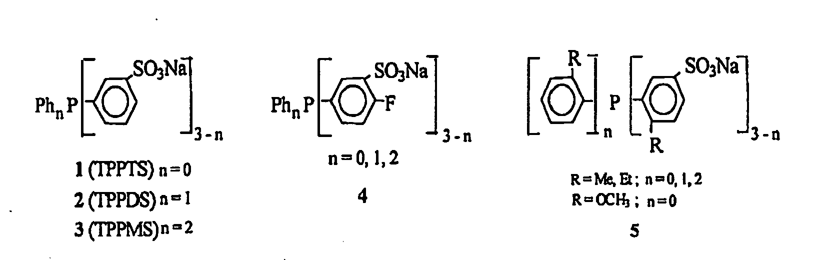

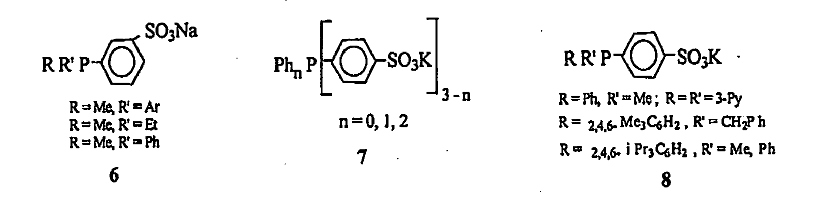

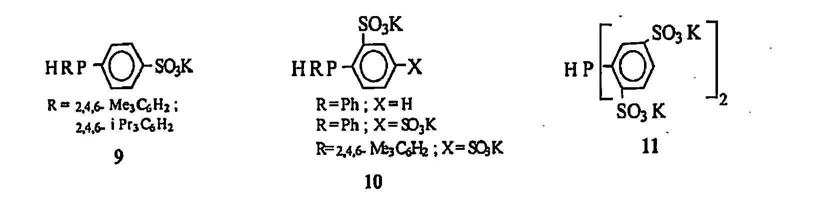

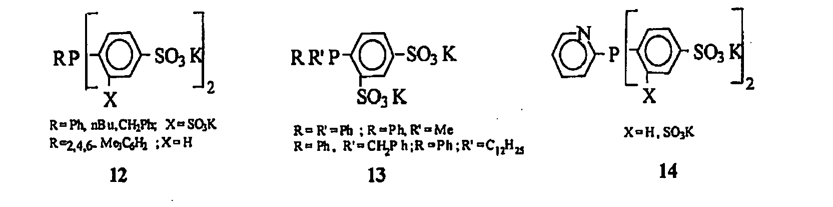

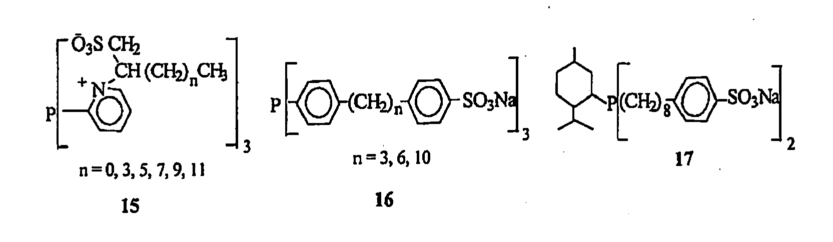

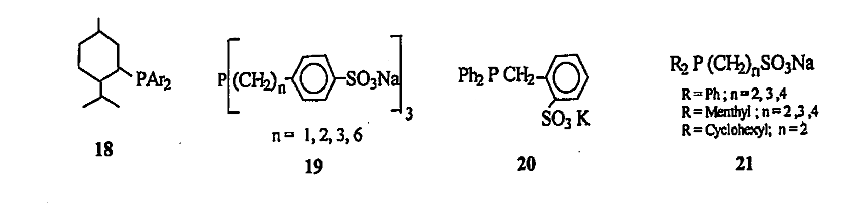

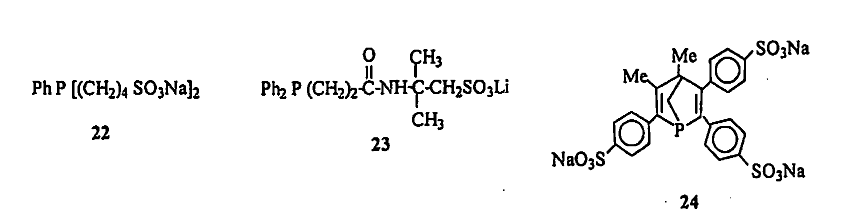

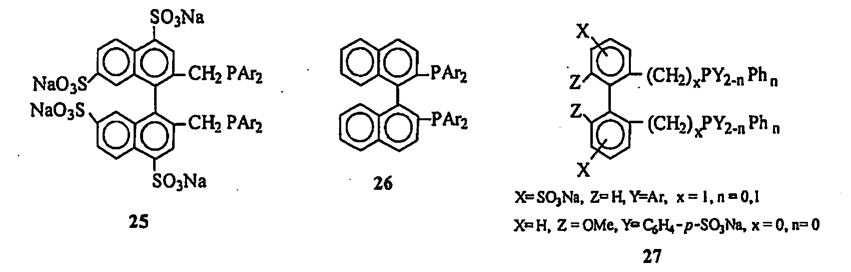

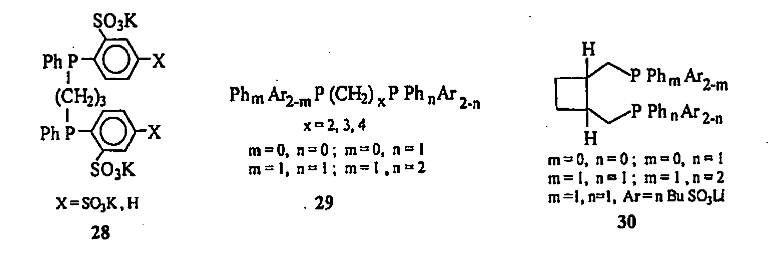

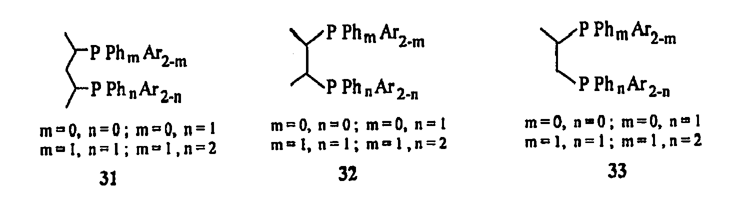

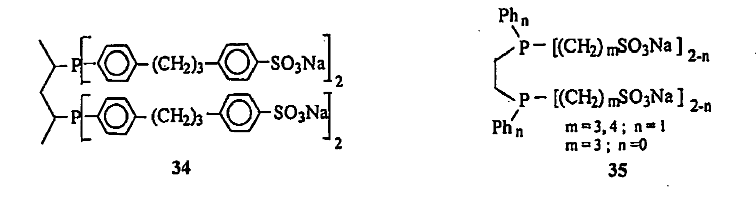

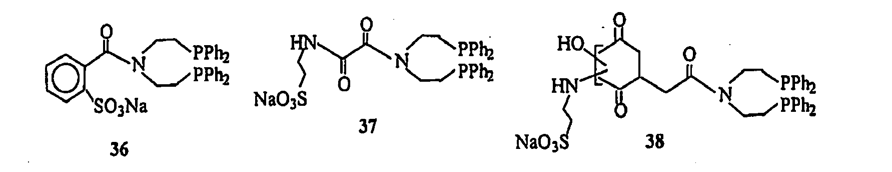

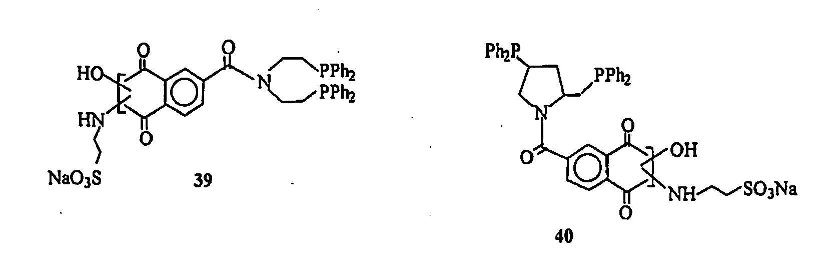

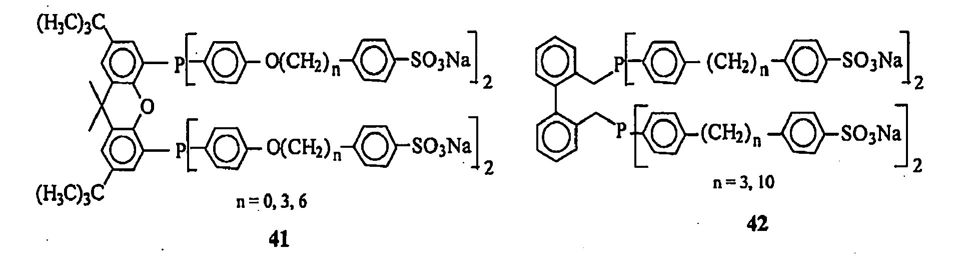

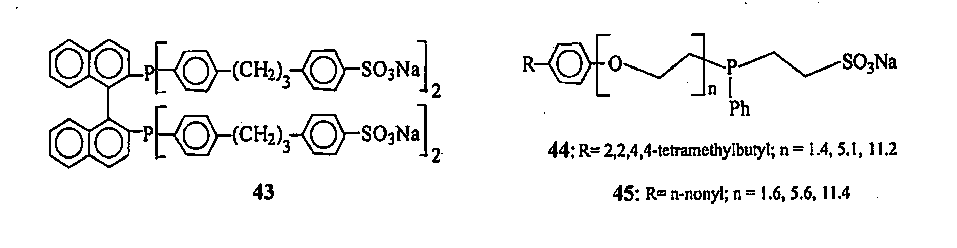

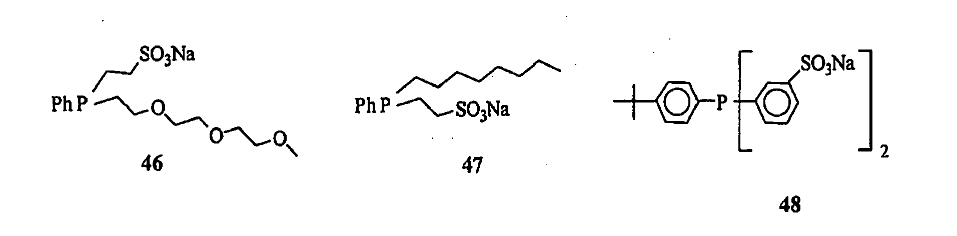

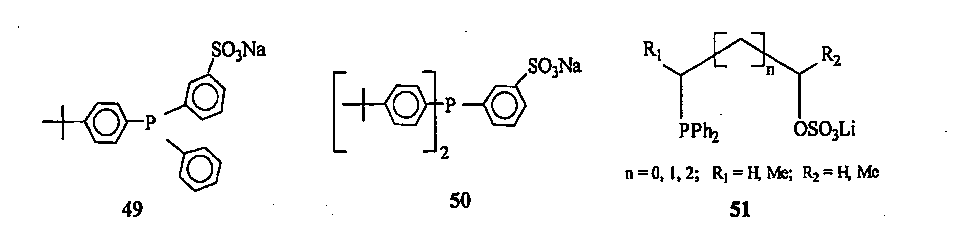

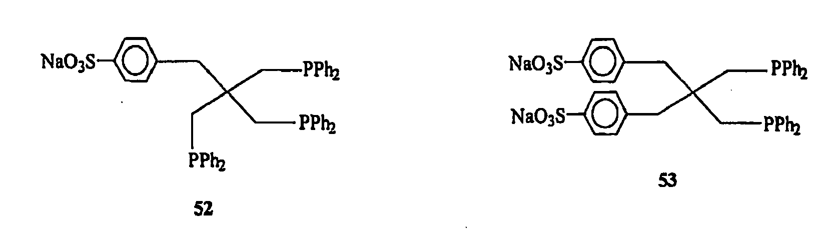

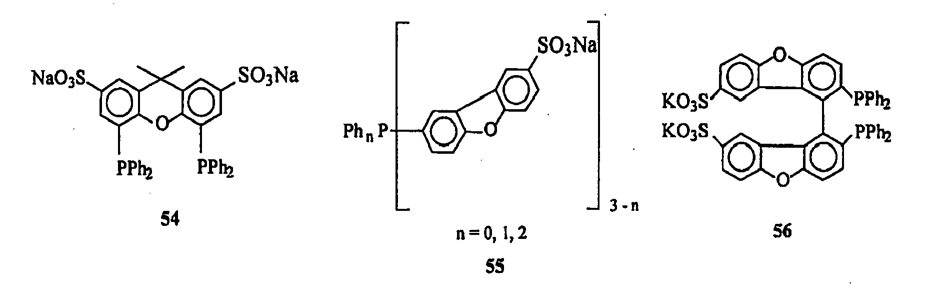

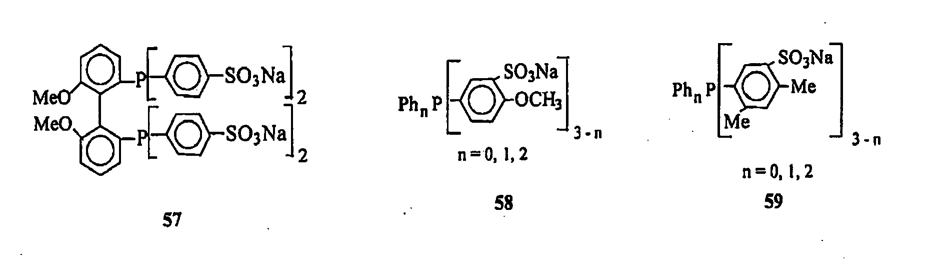

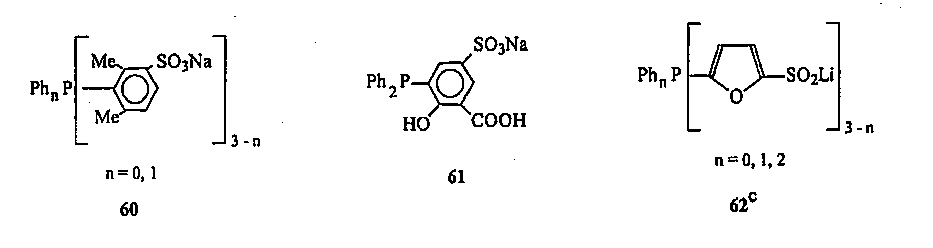

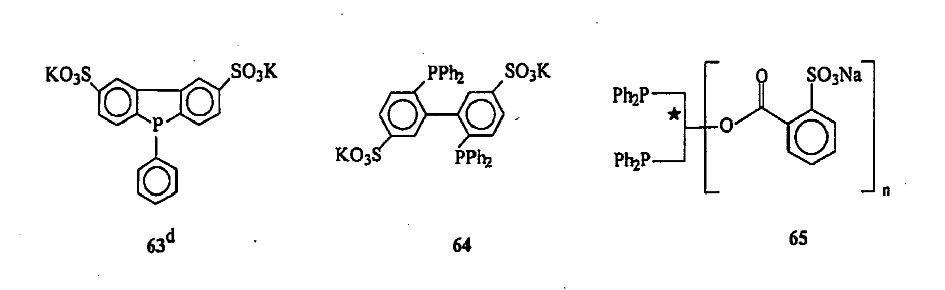

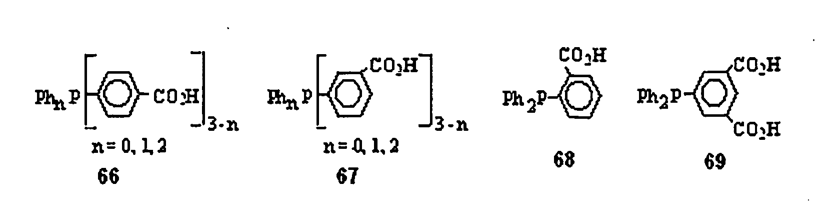

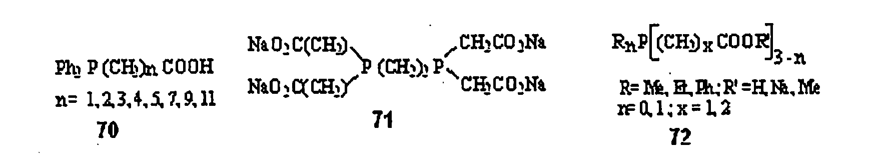

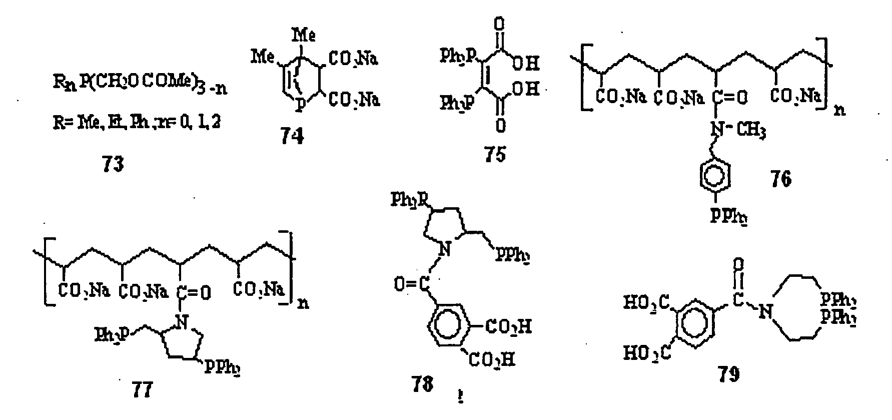

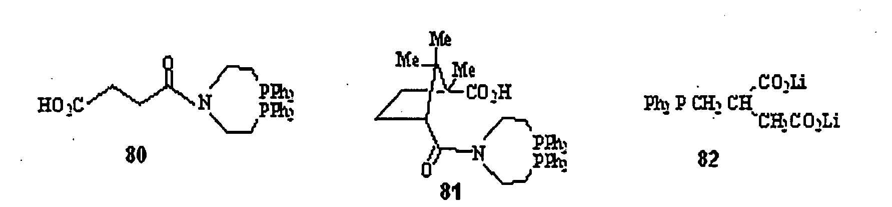

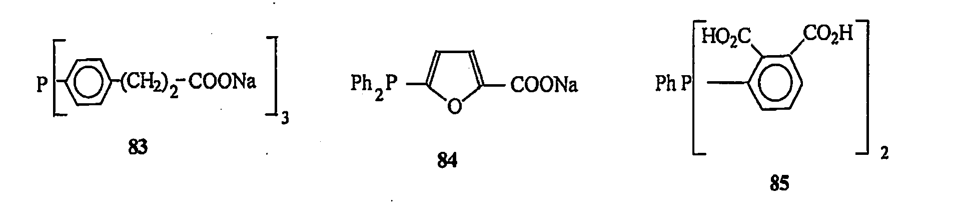

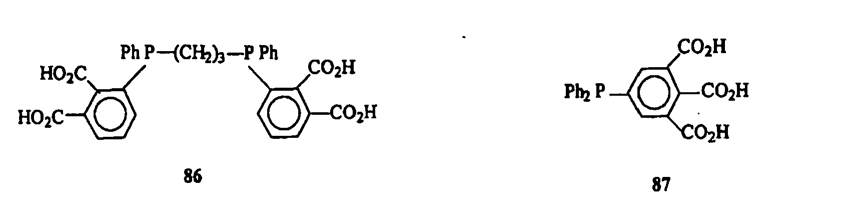

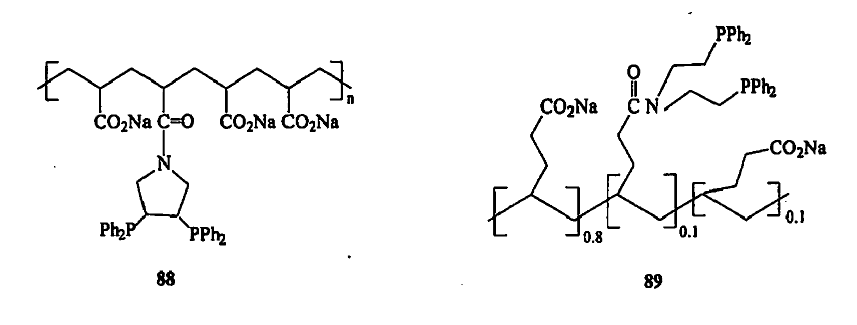

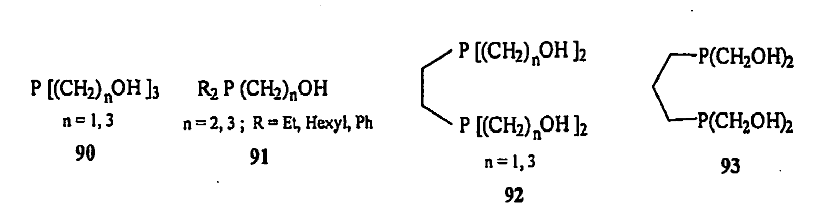

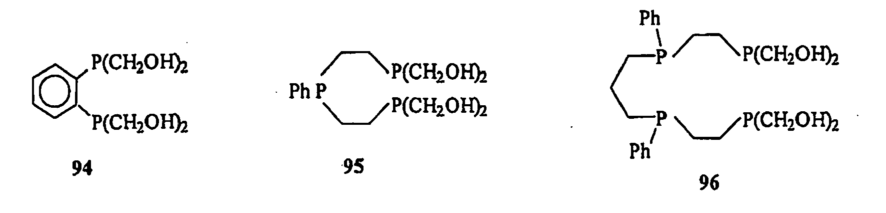

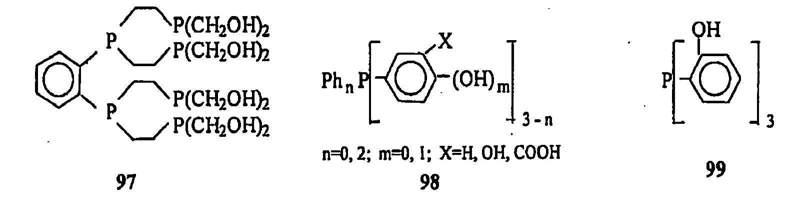

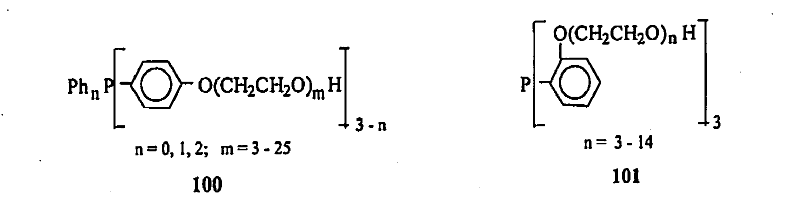

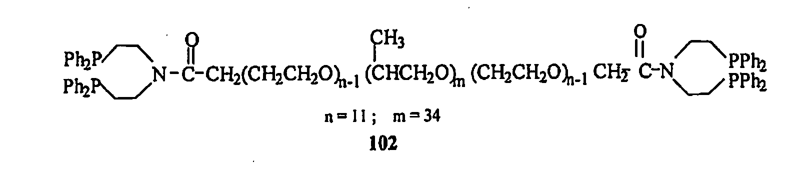

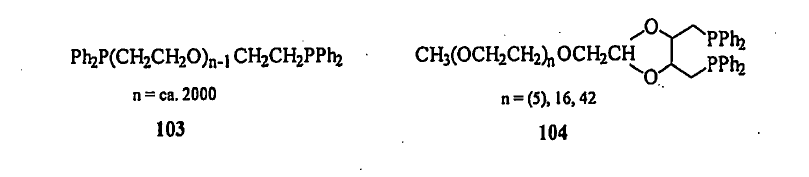

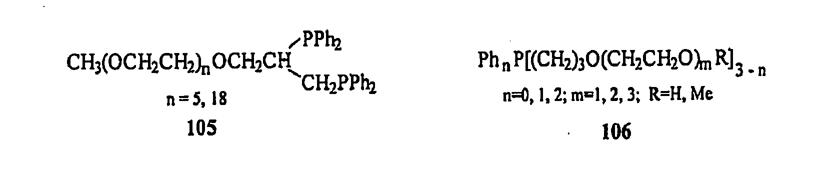

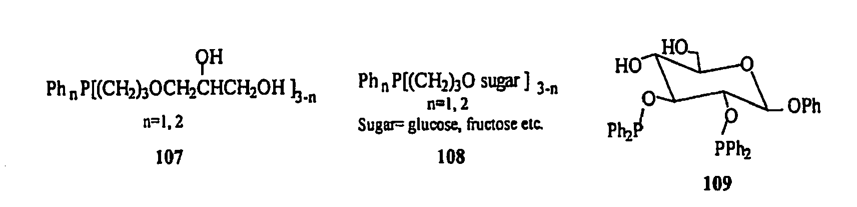

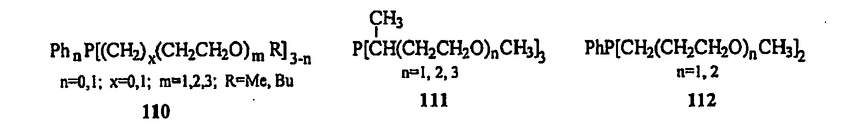

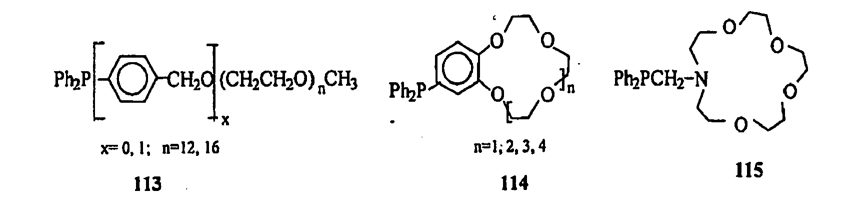

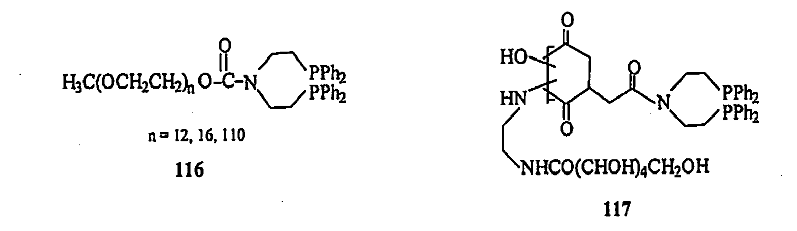

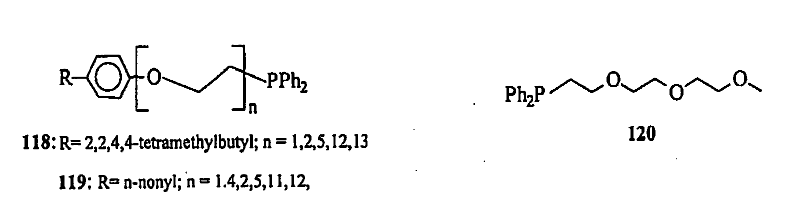

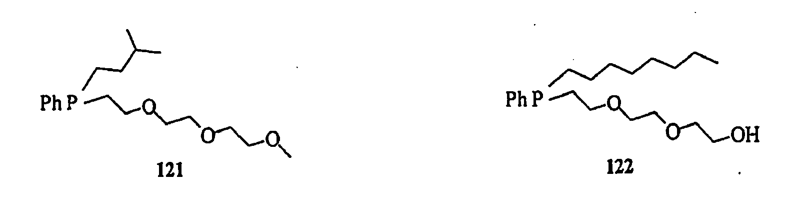

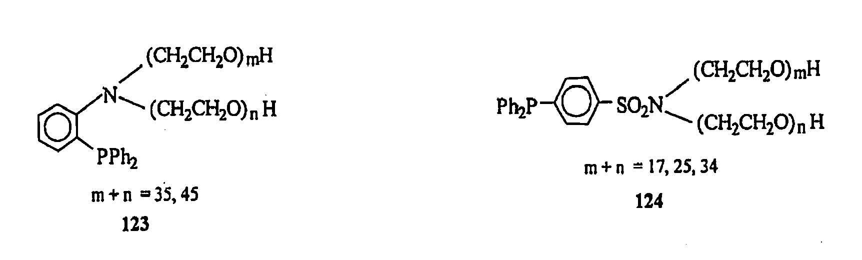

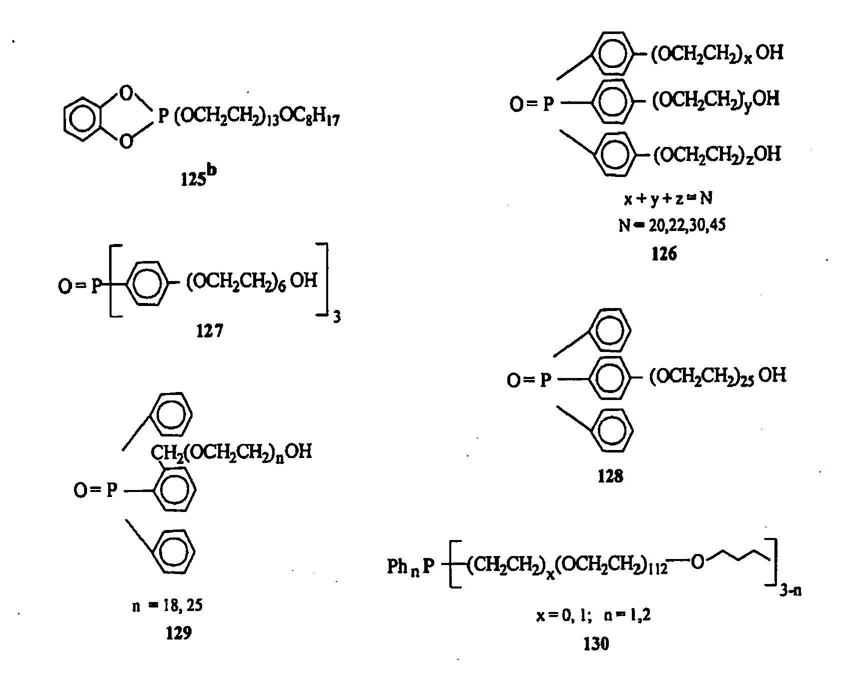

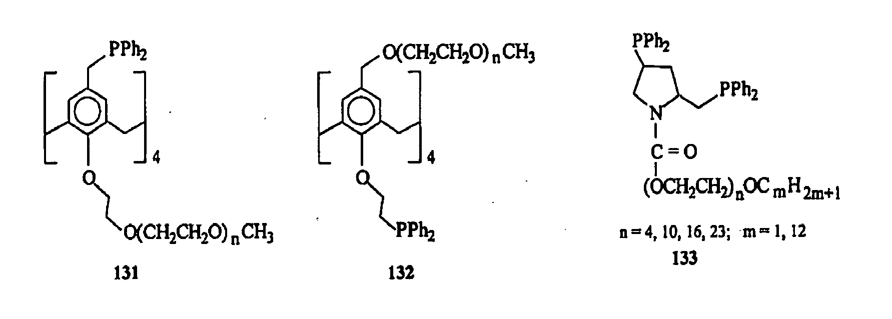

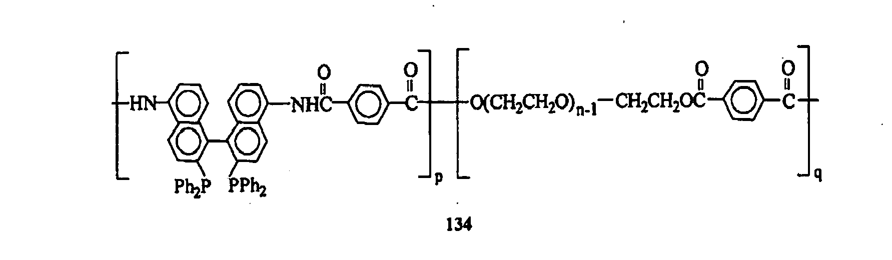

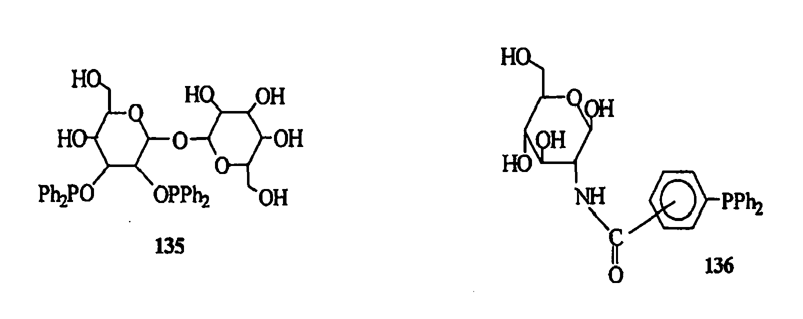

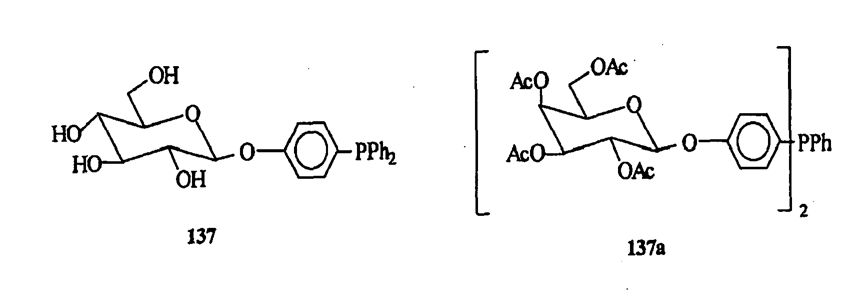

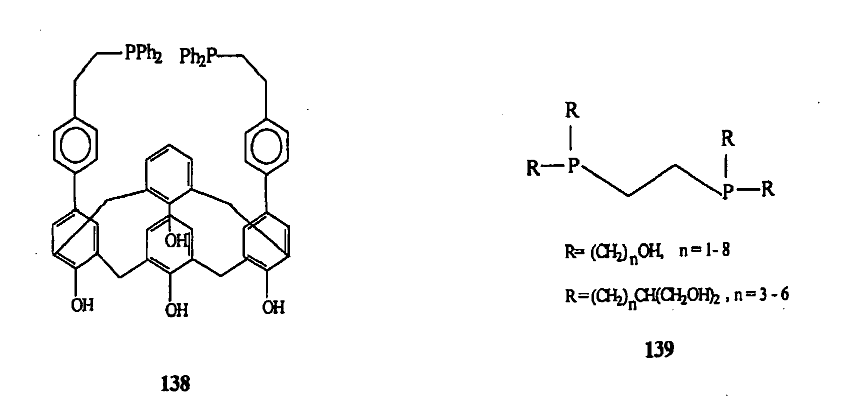

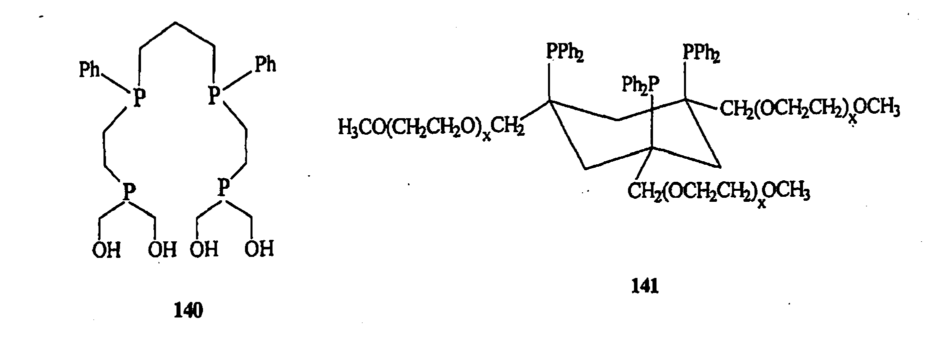

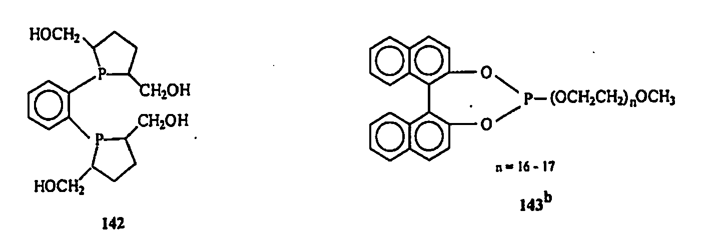

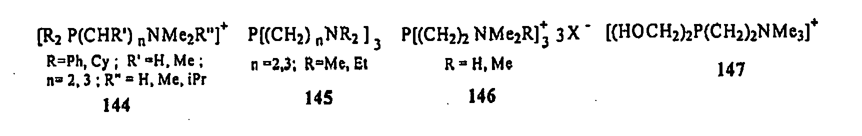

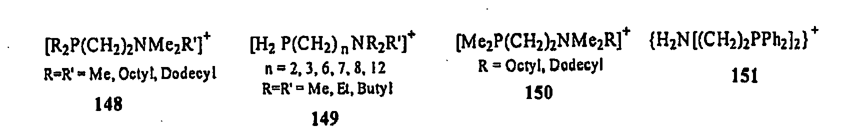

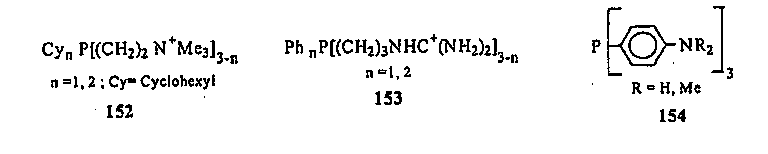

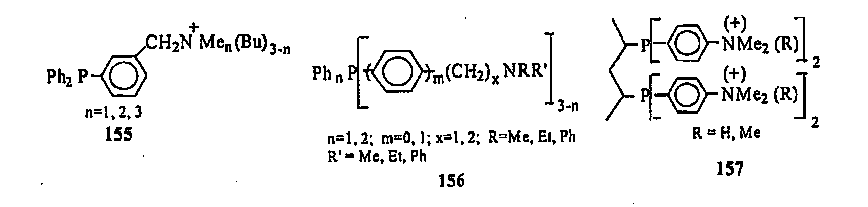

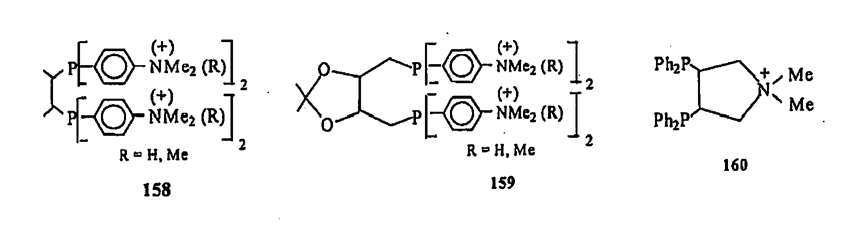

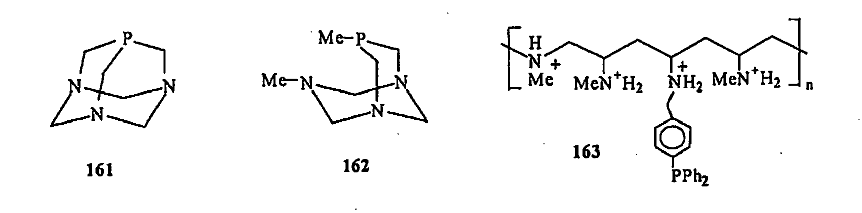

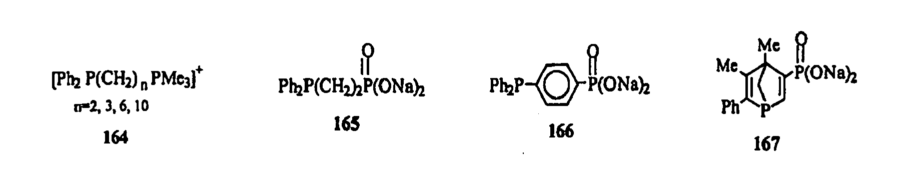

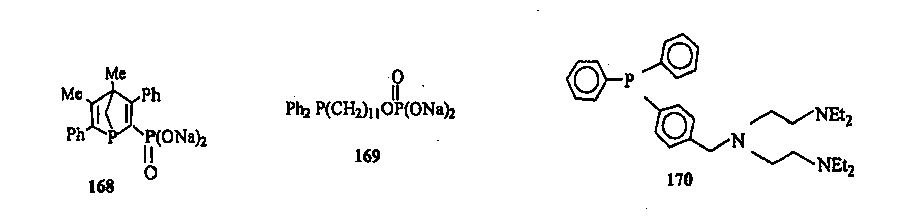

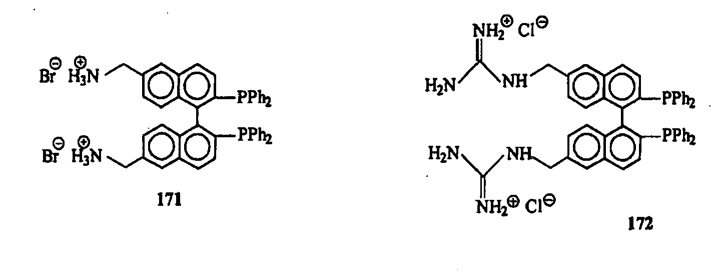

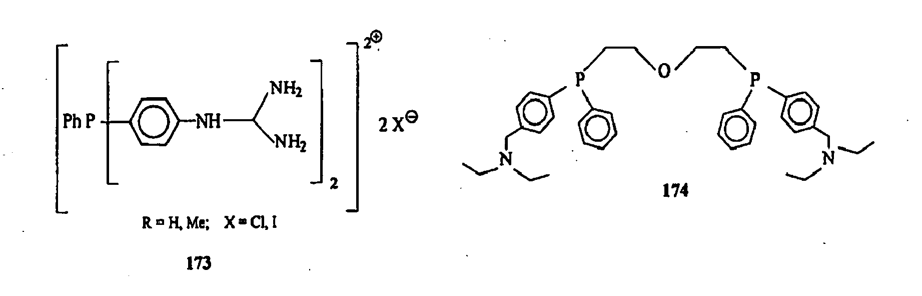

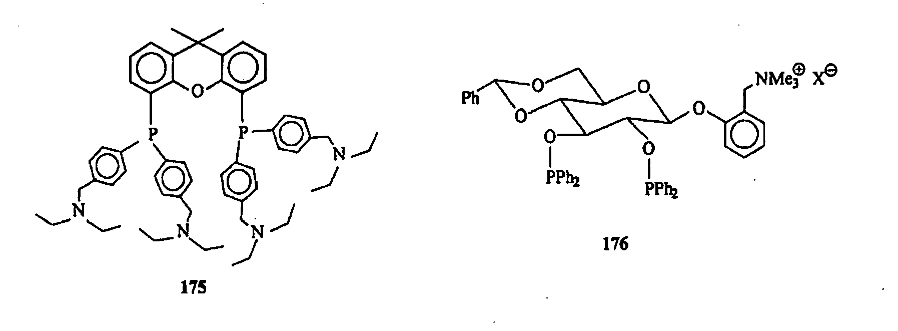

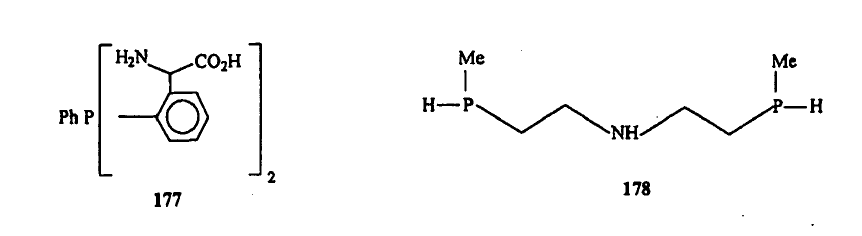

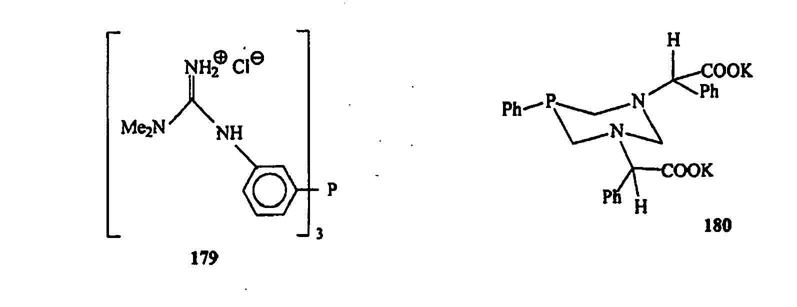

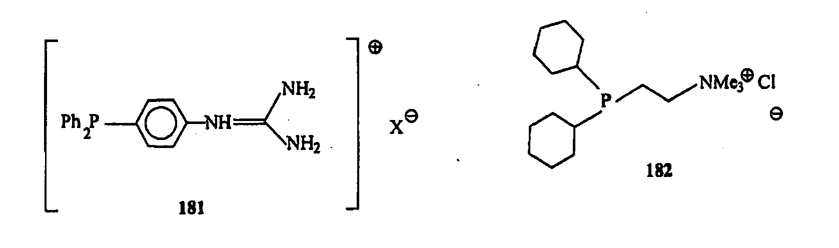

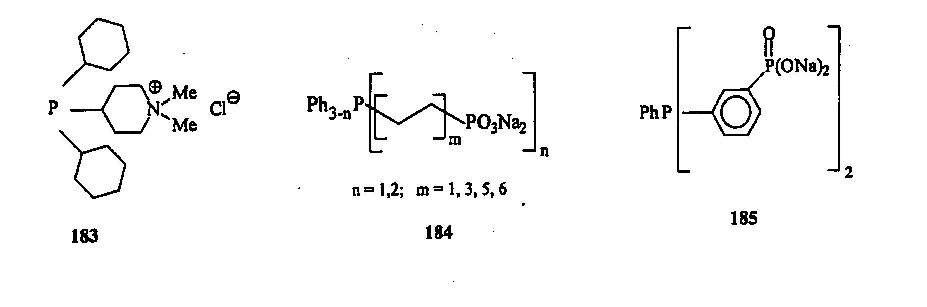

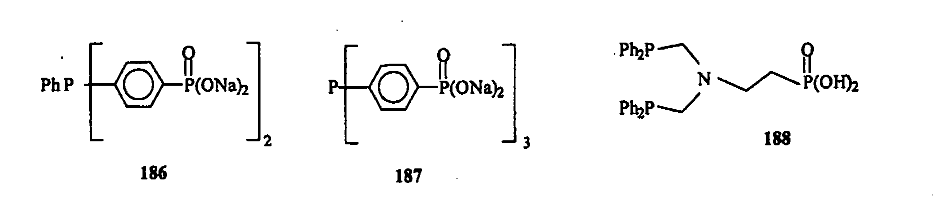

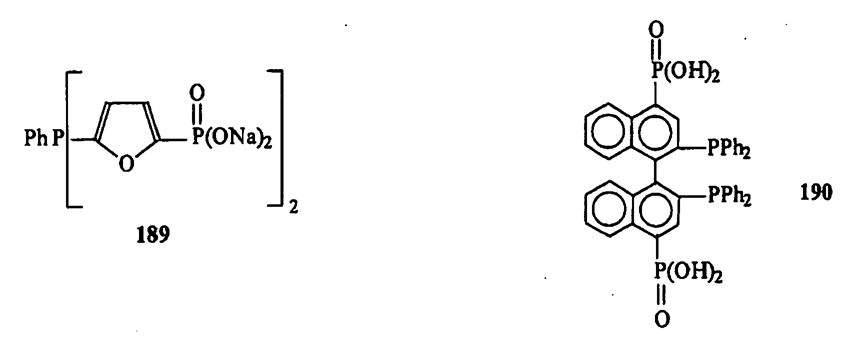

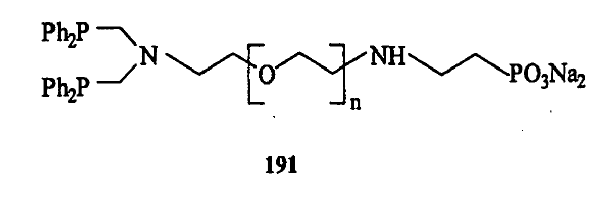

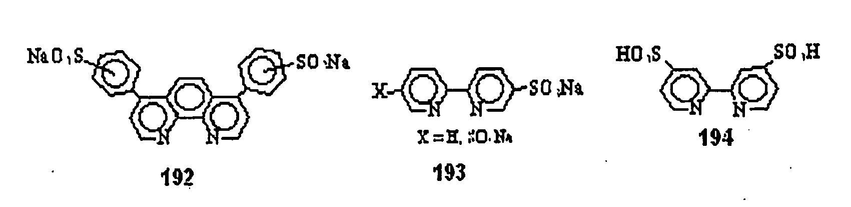

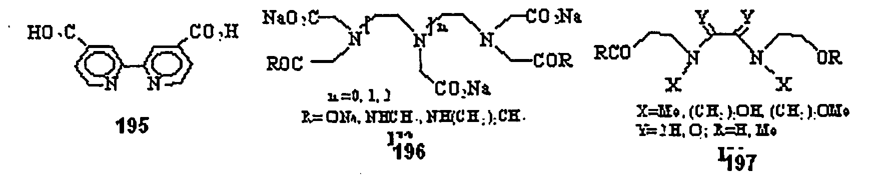

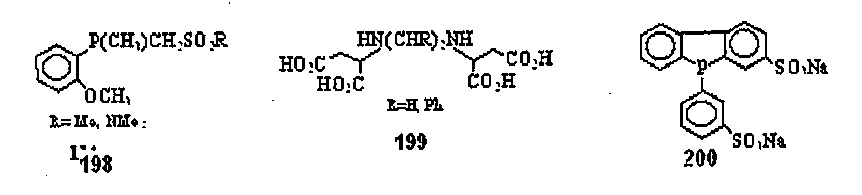

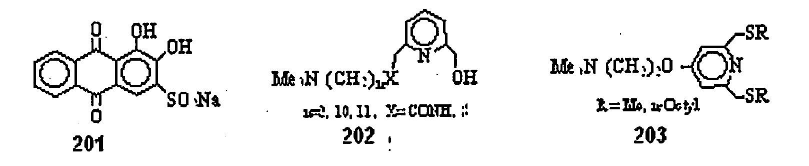

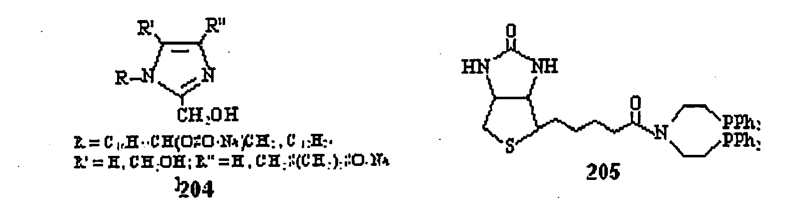

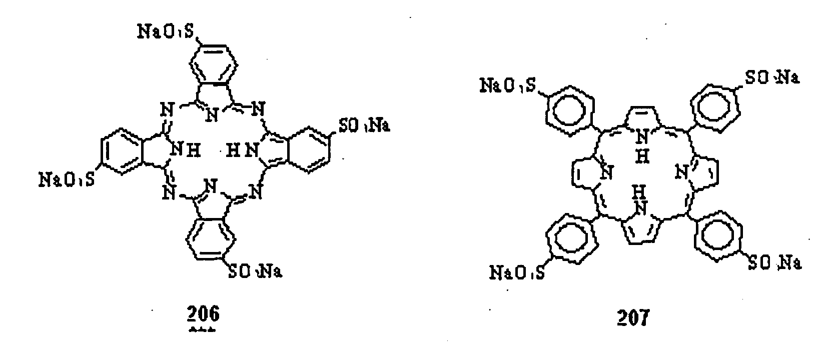

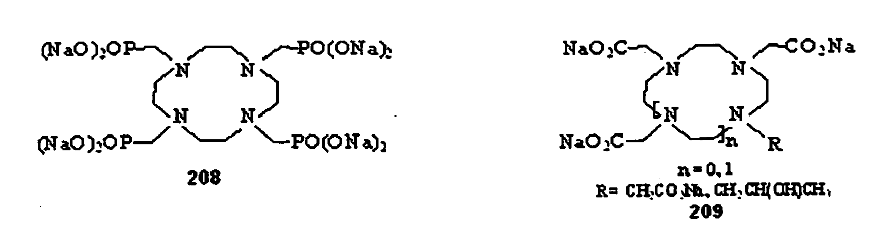

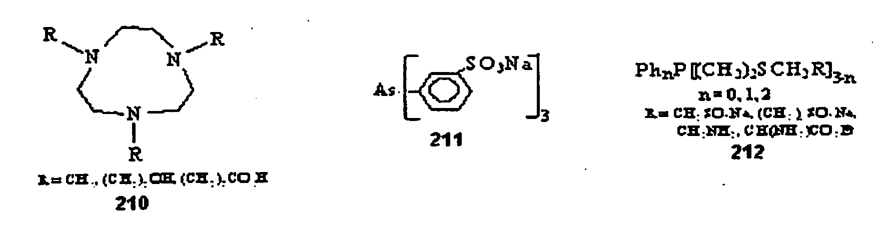

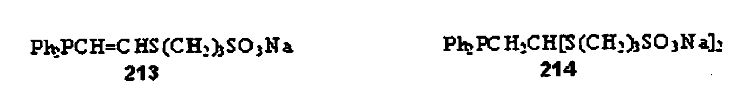

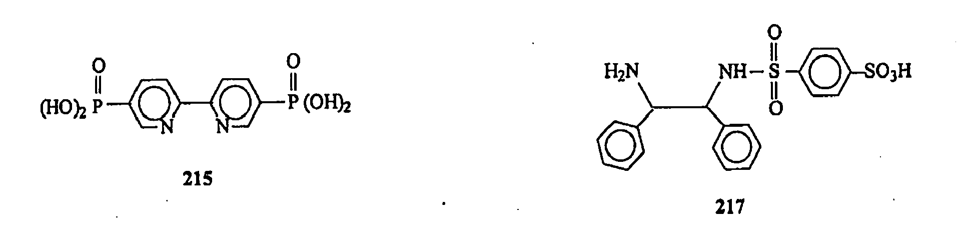

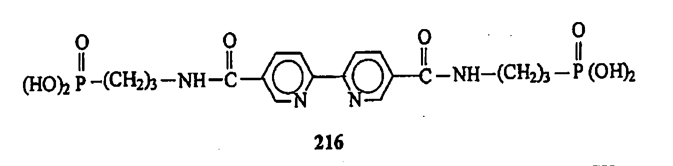

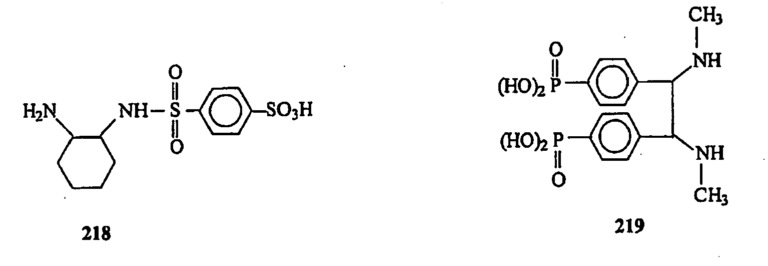

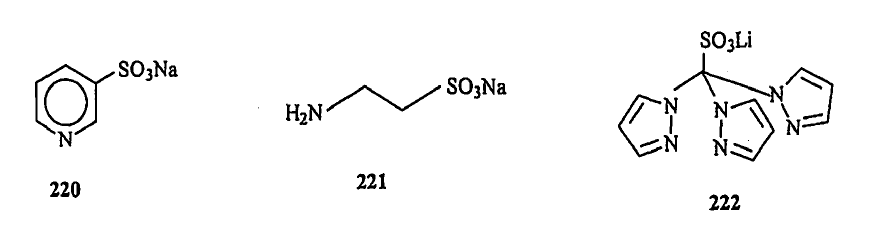

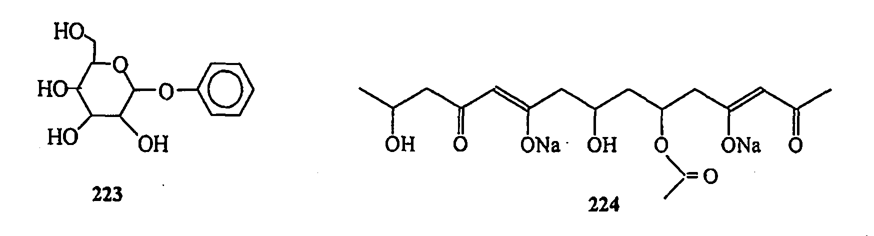

In a preferred embodiment of the present invention rhodium and/or ruthenium and/or palladium complexes have shown the highest activity and selectivity when combined with ligands of the trisulphonated triphenylphosphine (TPPTS) type. The catalyst is obtainable for example by simply mixing a watersoluble metal salt with the ligand, e.g. by preparing an aqueous solution of Rhodiumchloride or Rutheniumchloride or Palladiumchloride with TPPTS. In the following further details and preferred embodiments concerning the catalyst are given:
(i) molar ratio between Group VIII metal and hydrophilic ligand: about 1:1 to about 1:16, preferably about 1:2 to about 1:4;
(ii) content of Group VIII metal in the hydrogenation mixture: about 10 to about 500, preferably about 100 to about 340 ppm;
(iii) molar ratio C=C/Group VIII metal: about 500 to about 60,000, preferably about 100 to about 30,000 and more preferably about 5,000 to about 24,000.

The catalysts according to the present invention exhibit turnover frequencies in the range of about 200,000 h⁻¹. The catalytic behavior of the water soluble Wilkinson's catalyst, RhCl(TPPTS)₃, prepared separately, is comparable with the catalytic activity obtained by Rh/TPPTS complexes formed *in situ* from RhCl₃·3H₂O with TPPTS and H₂ in the hydrogenation of olefins under the same conditions in aqueous/organic two phase systems.

### Co-catalysts: cationic, zwitterionic (amphoteric), nonionic and anionic surfactants, ionic liquids and phase transfer catalysts

It has been found that the presence of cationic, zwitterionic (amphoteric), nonionic and anionic surfactants, ionic liquids (IL) and/or phase transfer catalysts (PTC) improves activity and selectivity of the catalyst and facilitates the separation of the complexes after the hydrogenation has taken place.

### Cationic and amphoteric surfactants

Typical examples for suitable cationic surfactants are the so-called tetraalkylammonium salts, which preferably follow general formula (I): 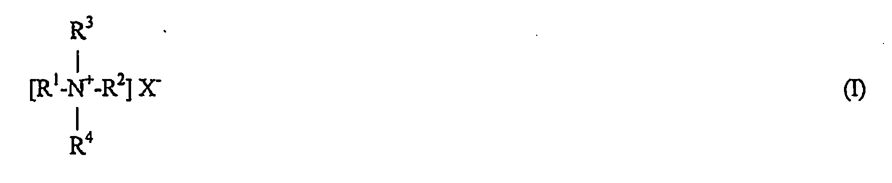 in which R¹, R², R³ and R⁴ independently from each other represent linear or branched, saturated or unsaturated aliphatic or hydroxyaliphatic radicals having 1 to 22 carbon atoms and X stands for chloride or bromide. Preferably at least two of these aliphatic radicals represent methyl groups, while at least one radical is a longer alkyl group having 8 to 18 carbon atoms. Typical examples are octyltrimethylammonium chloride (OTAC), dodecyltrimethylammonium chloride (DTAC), tetradecyltrimethylammonium chloride, cetyltrimethylammonium chloride (CTAC), cetylpyridinium chloride, octadecyltrimethylammonium chloride, distearyldimethylammonium chloride (DSDMAC). Other cationic surfactants of tetraalkylammonium salts include: tetrapentylammonium chloride, tetrahexylammonium chloride, tetradecylammonium chloride.

In the alternative, also cationic surfactants of the so-called esterquat type are useful, which can be derived from diethanol methylamine (DMA) and especially triethanol amine (TMA). The latter are preferably following general formula (II) 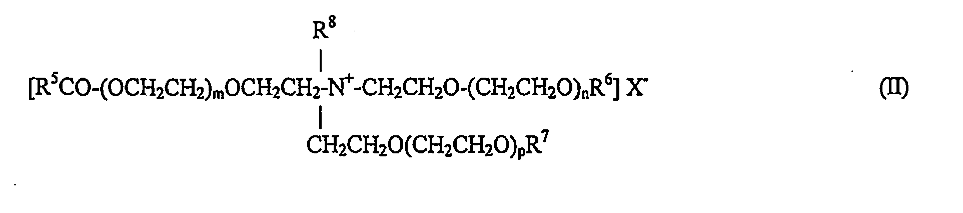 in which R⁵CO represents an acyl radical having 6 to 22, preferably 12 to 18 carbon atoms, R⁶ and R⁷ independently stand for hydrogen or R⁵CO, R⁸ means an alkyl radical having 1 to 4 carbon atoms or a (CH₂CH₂O)_{q}H-group, m, n and p represent in total 0 or an integer of 1 to 12, q represents numbers from 1 to 12 and X means halogenide, alkylsulphate or alkylphosphate. Typical and preferred examples are dicocoylmethylethoxymonium chloride and distearylmethylethoxymonium chloride which are sold under the trademark Dehyquart^{®} L80 or AU56 respectively.

Other suitable amphoteric surfactants among others are: 3-(N,N-dodecyldimethylammonium) propanesulphonate, 3-(NN-tetradecyldimethylammonium) propanesulphonate, 3-(N,N-cetyldimethylammonium) propanesulphonate, 3-(N,N-octadecyldimethylammonium) propanesulphonate, N,N-dimethy-loctylamino-N-oxide and N,N-dimethyl-decylamino-N-oxide.

### Non-ionic surfactants

Also non-ionic surfactants can be added as co-catalyst, including for example:
o products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
o C_{12/18} fatty acid monoesters and diesters of addition products of 1 to 30 mol ethylene oxide onto glycerol;
o alk(en)yl oligoglycosides;
o glycerol mono- and diesters and sorbitan mono- and diesters of saturated and unsaturated fatty acids containing 6 to 22 carbon atoms and ethylene oxide addition products thereof;
o addition products of 15 to 60 mol ethylene oxide onto castor oil and/or hydrogenated castor oil;
o polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
o addition products of 2 to 15 mol ethylene oxide onto castor oil and/or hydrogenated castor oil;
o partial esters based on linear, branched, unsaturated or saturated C_{6/22} fatty acids, ricinoleic acid and 12-hydroxystearic acid and glycerol, polyglycerol, pentaerythritol, dipentaerythritol, sugar alcohols (for example sorbitol), alkyl glucosides (for example methyl glucoside, butyl glucoside, lauryl glucoside) and polyglucosides (for example cellulose);
o mono-, di and trialkyl phosphates and mono-, di- and/or tri-PEG-alkyl phosphates and salts thereof;
o wool wax alcohols;
o polysiloxane/polyalkyl polyether copolymers and corresponding derivatives;
o mixed esters of pentaerythritol, fatty acids, citric acid and fatty alcohol and/or mixed esters of C₆₋₂₂ fatty acids, methyl glucose and polyols, preferably glycerol or polyglycerol,
o polyalkylene glycols and
o glycerol carbonate.

The addition products of ethylene oxide and/or propylene oxide onto fatty alcohols, fatty acids, alkylphenols, alk(en)yl oligoglycosides, glycerol mono- and diesters and sorbitan mono-and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations. The preferred emulsifiers are described in more detail as follows:
**Alk(en)yl** oligoglycosides. Alkyl or alkenyl oligoglycosides may be derived from aldoses or ketoses containing 5 or 6 carbon atoms, preferably glucose. Accordingly, the preferred alkyl and/or alkenyl oligoglycosides are alkyl or alkenyl oligoglucosides. These materials are also known generically as "alkyl polyglycosides" (APG). The alk(en)yl oligoglycosides according to the invention correspond to formula (III):

   R⁹O[G]ₚ (III)

   wherein R⁹ is an alkyl or alkenyl radical having from 6 to 22 carbon atoms, G is a sugar unit having 5 or 6 carbon atoms and p is a number from 1 to 10. The index p in general formula (III) indicates the degree of oligomerisation (DP degree), i.e. the distribution of mono- and oligoglycosides, and is a number of 1 to 10. Whereas p in a given compound must always be an integer and, above all, may assume a value of 1 to 6, the value p for a certain alkyl oligoglycoside is an analytically determined calculated quantity which is mostly a broken number. Alk(en)yl oligoglycosides having an average degree of oligomerisation p of 1.1 to 3.0 are preferably used. Alk(en)yl oligoglycosides having a degree of oligomerisation below 1.7 and, more particularly, between 1.2 and 1.4 are preferred from the applicational point of view. The alkyl or alkenyl radical R⁹ may be derived from primary alcohols containing 4 to 22 and preferably 8 to 16 carbon atoms. Typical examples are butanol, caproic alcohol, caprylic alcohol, capric alcohol, undecyl alcohol, lauryl alcohol, myristyl alcohol, cetyl alcohol, palmitoleyl alcohol, stearyl alcohol, isostearyl alcohol, oleyl alcohol, elaidyl alcohol, petroselinyl alcohol, arachyl alcohol, gadoleyl alcohol, behenyl alcohol, erucyl alcohol and technical mixtures thereof such as are formed, for example, in the hydrogenation of technical fatty acid methyl esters or in the hydrogenation of aldehydes from Roelen's oxo synthesis. Alkyl oligoglucosides based on hydrogenated C₈-C₁₆ coconut oil alcohol having a DP of 1 to 3 are preferred.
***Partial glycerides.*** Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.
*Sorbitan esters.* Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.
*Polyglycerol esters.* Typical examples of suitable polyglycerol esters are Polyglyceryl-2 Dipolyhydroxystearate (Dehymuls^{®} PGPH), Polyglycerin-3-Diisostearate (Lameform^{®} TGI], Polyglyceryl-4 Isostearate (Isolan^{®} GI 34), Polyglyceryl-3 Oleate, Diisostearoyl Polyglyceryl-3 Diisostearate (Isolan® PDI), Polyglyceryl-3 Methylglucose Distearate (Tego Care^{®} 450), Polyglyceryl-3 Beeswax (Cera Bellina^{®}, Polyglyceryl-4 Caprate (Polyglycerol Caprate T2010/90), Polyglyceryl-3 Cetyl Ether (Chimexane^{®} NL), Polyglyceryl-3 Distearate (Cremophor® GS 32) and Polyglyceryl Polyricinoleate (Admul^{®} WOL 1403), Polyglyceryl Dimerate Isostearate and mixtures thereof. Examples of other suitable polyolesters are the mono-, di-and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

In particular preferred examples for suitable nonionic surfactants of Brij-type are among others the following systems: 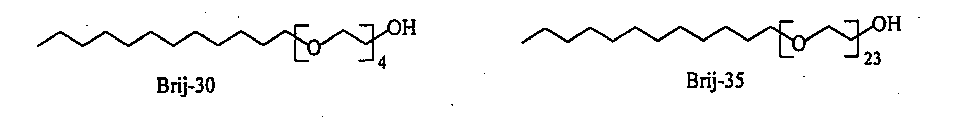 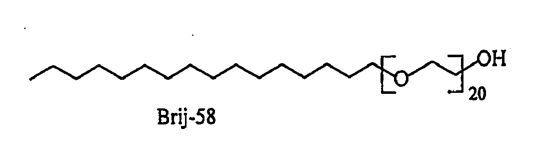 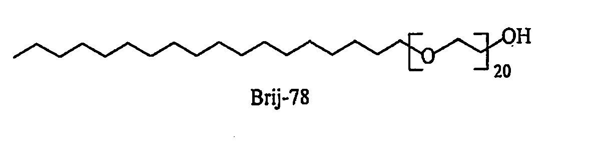 and of Triton X - type such as: 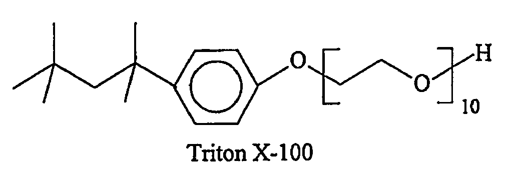 as well as Triton X-114, Triton X-305 and Triton X-405.

Anionic surfactants suitable as cocatalysts in this biphasic catalytic hydrogenation reaction are among others: sodium dodecylsulphate (SDS): 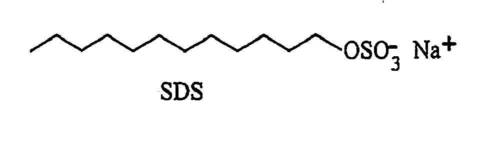 dodecylbenzonesulphonate (DBS), sodium 1-heptanesulphonate, sodium I-octanesulphonate, sodium 1-nonanesulphonate, sodium 1-decanesulphonate and salts of linear alkylbenzenesulphonate (LABS).

Suitable phase transfer catalysts also useful as co-catalysts are summarised in J.Am.Chem.Soc., 93, p195f, (1971). Typical examples are tetraalkylammonium salts with short alkyl groups, benzyltrialkylammonium salts, tetraalkylphosphonium salts, benzyltrialkylphosphonium salts and their mixtures. In preferred embodiments of the present invention tetra-n-butylammonium, tri-n-butylmethylammonium, benzyltriethylammonium, tetra-n-butylphosphonium, tri-n-butylmethylphosphonium, benzyltriethylphosphonium in form of their chlorides, bromides or hydrogensulphates are used.

The molar ratio between the co-catalyst and the ligand is typically about 50:1 to 0.01:1. and preferably about 2:1 to about 0.3:1.

### Hydrogenation process

The process according to the present invention follows the reactions which are shown below:

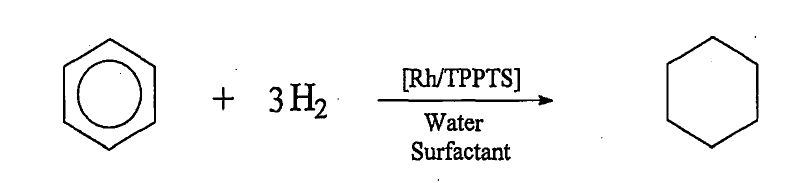

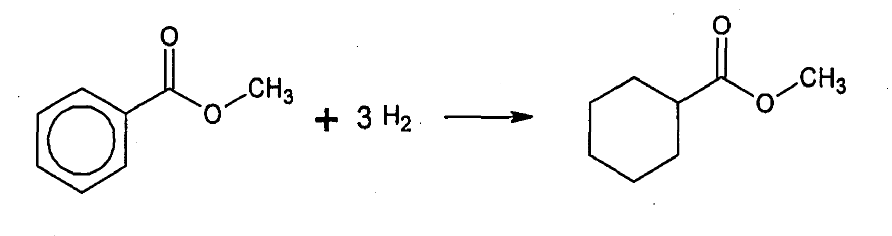

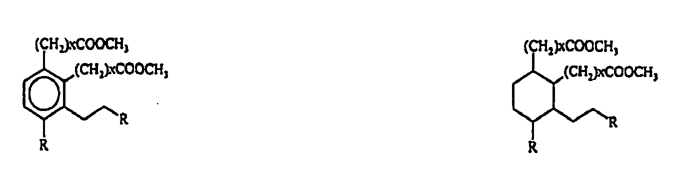

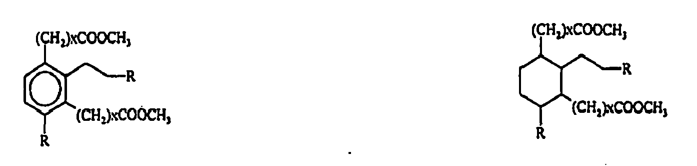

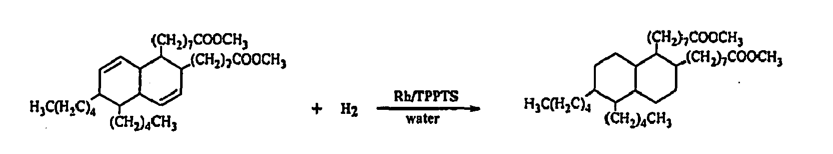

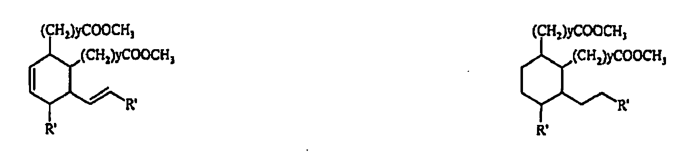

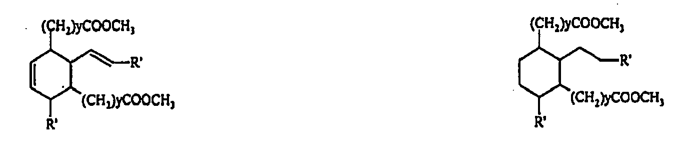

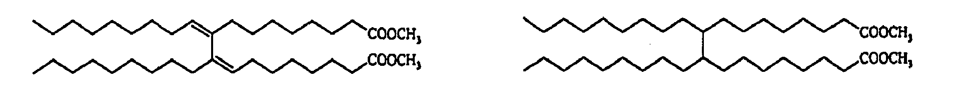

In the first step the catalyst is prepared, for example by simply dissolving the Group VIII metal salt (e.g. RhCl₃ 3H₂O) and the hydrophilic ligand (e.g. the sodium salt of TPPTS) in demineralised and deaerated water. The aqueous catalyst system is mixed with the benzene or methyl benzoate or dimer fatty acid methyl ester mixture to result in a two-phase system formed by the aqueous catalyst solution and the benzene or methyl benzoate or dimer fatty acid methyl ester mixture. The ratio between the volume of the aqueous and the organic phase is typically about 50 to about 0.5 and preferably about 0.7. The mixture comprising the benzene or methyl benzoate or dimer fatty acid methyl ester mixture and the catalyst is transferred into a stirred autoclave purged with argon and after a number of pressurising-depressurising cycles with hydrogen in order to remove all traces of oxygen. Then the reactor is heated up to 80 to 130°C, preferably 100 to 120 °C at kept there for a reaction time of 5 to 120, preferably 10 to 60 minutes. During this time the pressure rises up to 10 to 90 bar. Once the reaction has been completed the mixtures is cooled to room temperature and depressurised. The upper organic layer comprising the hydrogenation product is separated from the lower aqueous layer and analyzed. The aqueous layer containing the catalyst is recycled. GC analyses of the product of the hydrogenation of benzene were run on a Shimadzu GC-14B equipped with a flame ionization detector (FID) and a HP-Innowax capillary column (30m x 0.251mm i.d. x 0.50µm film thickness). Carrier gas was N₂ at 50 kPa. The oven temperature was 85°C. The injector and detector temperatures were set at 240°C. GC analyses of the products of the hydrogenation of methyl benzoate were run on a Shimadzu GC-14B equipped with a flame ionization detector (FID) and a HP-Innowax capillary column (30m x 0.251mm i.d. x 0.50µm film thickness). Carrier gas was N₂ at 140 kPa. The oven temperature was initially at 120°C for 0 min and then increased to 240°C at 3°C/min. The injector and detector temperatures were both set at 240°C.

### Industrial application

As explained above, the present invention is extremely useful to provide hydrogenated cyclic compounds by selective hydrogenation of the respective aromatic starting materials. The hydrogenated aromatics obtained according to the process of the present invention are useful for example for the manufacture of nylon 6 and nylon 6.6 polyamides, for the removal of benzene of gasoline and of other aromatics contained in diesel fuel, for the manufacture of improved stability of specialty polyamide oligomers (Versamids) coatings, epoxy hardeners, lubricants and cosmetics based on dimer fatty acid alkyl esters or alcohols, and for the inhibition of oxidative yellowing of paper made from mechanical pulps.

### Examples

### Examples 1 to 6

### Selective hydrogenation of benzene to cyclohexane - Influence of temperature

The following benzene hydrogenation examples were conducted at changing temperature. The reaction conditions were the following:
○ P_{H2} = 30 bar
○ Hydrogenation time = 10 min
○ 2.6 mg (0.01 mmol) RhCl₃· 3H₂O; 18.5mg (0.03 mmol) TPPTS (P/Rh molar ratio= 3)
○ 2.34 g (30 mmol) benzene (C=C/Rh molar ratio = 9000)
○ 10 g deaerated distilled demineralized water
○ [Rh] = 100 ppm; stirring rate = 670 rpm

Additional details of the hydrogenation and the results of the trials are compiled in Table 1:

**Table 1**

| Biphasic hydrogenation of benzene catalyzed by Rh/TPPTS complexes at different temperatures | | | | | | |
|---|---|---|---|---|---|---|
| **Ex.** | **Catalyst Precursor** | **C=C/Rh molar ratio** | **T (°C)** | **Conversion** **(mol%)** | **Selectivity of cyclohexane(mol%)** | **TOF^{a}** **(h⁻¹)** |
| 1 | RhCl₃·3H₂O/TPPTS | 9000 | 80 | 47 | 100 | 25170 |
| 2 | RhCl₃·3H₂O/TPPTS | 9000 | 90 | 56 | 100 | 30150 |
| 3 | RhCl₃·3H₂O/TPPTS | 9000 | 100 | 86 | 100 | 46560 |
| 4 | RhCl₃·3H₂O/TPPTS | 9000 | 110 | 91 | 100 | 49260 |
| 5 | RhCl₃·3H₂O/TPPTS | 9000 | 120 | 89 | 100 | 48100 |
| 6 | RhCl₃·3H₂O/TPPTS | 9000 | 130 | 98 | 100 | 53010 |

| | | | | | | |
|---|---|---|---|---|---|---|
| ^{a} Defined as mole of hydrogenated C=C units of benzene per mole of Rh per hour. | | | | | | |

### Examples 7 - 12

### Selective hydrogenation of benzene to cyclohexane - Influence of TPPTS/Rh molar ratio

The following benzene hydrogenation examples were conducted at changing TPPTS/Rh molar ratio. The reaction conditions were the following:
○ T = 120°C
○ P_{H2} = 50 bar
○ Hydrogenation time =10 min
○ 2.63 mg (0.01 mmol) RhCl₃· 3H₂O
○ 3.91 g (50 mmol) benzene (C=C/Rh molar ratio = 15000)
○ 10 g deaerated distilled demineralized water
○ [Rh] =100 ppm; stirring rate = 670 rpm

Additional details of the hydrogenation and the results of the trials are compiled in Table 2:

**Table 2**

| Biphasic hydrogenation of benzene catalyzed by Rh/TPPTS complexes at different TPPTS/Rh molar ratios | | | | | | |
|---|---|---|---|---|---|---|
| **Ex.** | **Catalyst Precursor** | **C=C/Rbh molar ratio** | **TPPTS/Rh molar ratio** | **Conversion** **(mol %)** | **Selectivity of cyclohexane(mol %)** | **TOF^{a}** **(h⁻¹)** |
| 7 | RhCl₃·3H₂O/TPPTS | 15000 | 3 | 77 | 100 | 68970 |
| 8 | RhCl₃·3H₂O/TPPTS | 15000 | 4 | 88 | 100 | 79050 |
| 9 | RhCl₃·3H₂O/TPPTS | 15000 | 5 | 87 | 100 | 78246 |
| 10 | RhCl₃·3H₂O/TPPTS | 15000 | 8 | 83 | 100 | 74790 |
| 11 | RhCl₃·3H₂O/TPPTS | 15000 | 12 | 68 | 100 | 60720 |
| 12 | RhCl₃·3H₂O/TPPTS | 15000 | 16 | 53 | 100 | 47820 |

| | | | | | | |
|---|---|---|---|---|---|---|
| ^{a}Defined as mole of hydrogenated C=C units of benzene per mole of Rh per hour. | | | | | | |

### Examples 13 - 17

### Selective hydrogenation of benzene to cyclohexane - Influence of hydrogen partial pressure

The following benzene hydrogenation examples were conducted under different hydrogen partial pressure. The reaction conditions were the following:
○ T =120° C
○ Hydrogenation time =10 min
○ 2.63mg (0.01 mmol) RhCl₃·3H₂O
○ 18.5 mg (0.03 mmol) TPPTS (P/Rh molar ratio = 3)
○ 3.91 g (50 mmol) benzene (C=C/Rh molar ratio = 15000)
○ 10 g deaerated distilled demineralized water
○ [Rh] =100 ppm
○ Stirring rate = 670 rpm

Additional details of the hydrogenation and the results of the trials are compiled in Table 3:

**Table 3**

| Biphasic hydrogenation of benzene catalyzed by Rh/TPPTS complexes at different hydrogen pressures | | | | | | |
|---|---|---|---|---|---|---|
| **Ex.** | **Catalyst Precursor** | **C=C/Rh molar ratio** | **P_{HZ}** **(bar)** | **Conversion** **(mol %)** | **Selectivity of cyclohexane** **(mol %)** | **TOF^{a}** (h**⁻¹)** |
| 13 | RhCl₃·3H₂O/TPPTS | 15000 | 30 | 62 | 100 | 55920 |
| 14 | RhCl₃·3H₂O/TPPTS | 15000 | 50 | 77 | 100 | 68970 |
| 15 | RhCl₃·3H₂O/TPPTS | 15000 | 60 | 87 | 100 | 78720 |
| 16 | RhCl₃·3H₂O/TPPTS | 15000 | 70 | 92 | 100 | 83190 |
| 17 | RhCl₃·3H₂O/TPPTS | 15000 | 80 | 96 | 100 | 86790 |

| | | | | | | |
|---|---|---|---|---|---|---|
| ^{a}Defined as mole of hydrogenated C=C units of benzene per mole of Rh per hour. | | | | | | |

### Examples 18 - 21

### Selective hydrogenation of benzene to cyclohexane - Influence of the presence and the nature of added surfactant

The following benzene hydrogenation examples were conducted in the absence and in the presence of cationic and non-ionic surfactants. The reaction conditions were the following:
○ T = 130° C
○ P_{H2} = 80 bar
○ Hydrogenation time = 5 min
○ 2.63 mg (0.01 mmol) RhCl₃· 3H₂O
○ Stirring rate = 670 rpm

Additional details of the hydrogenation and the results of the trials are compiled in Table 4:

**Table 4**

| Biphasic hydrogenation of benzene catalyzed by Rh/TPPTS complexes in the absence and presence of cationic and non-ionic surfactants | | | | | | | | |
|---|---|---|---|---|---|---|---|---|
| **Ex.** | **Catalyst Precursor** | **P/Rh molar ratio** | **C=C/Rh molar ratio** | **Surfactant/ TPPTS molar ratio** | **H₂O** **(g)** | **Conversion** **(mol %)** | **Selectivity of cyclohexane** **(mol %)** | **TOF^{a}** **(h⁻¹)** |
| 18 | RhCl₃·3H₂O/ TPPTS | 3 | 36000 | - | 5 | 30 | 100 | 128160 |
| 19 | RhCl₃·3H₂O/ TPPTS | 4 | 36000 | DTAC^{b} /TPPTS= 2 | 5 | 23 | 100 | 99750 |
| 20 | RhCl₃·3H₂O/ TPPTS | 4 | 30000 | Brij35^{c} /TPPTS =2 | 3 | 52 | 100 | 187530 |
| 21 | RhCl₃·3H₂O/ TPPTS | 4 | 30000 | Brij30^{d} /TPPTS =2 | 3 | 57 | 100 | 204400 |

| | | | | | | | | |
|---|---|---|---|---|---|---|---|---|
| ^{a} Defined as mole of hydrogenated C=C units of benzene per mole of Rh per hour. ^{b} DTAC = dodecyltrimethylammonium chloride. ^{c} Brij 35 = polyoxyethylene(23)lauryl ether. ^{d} Brij 30 = polyoxyethylene(4)lauryl ether. | | | | | | | | |

### Examples 22 - 24

### Selective hydrogenation of benzene to cyclohexane - Influence of Brij 35/TPPTS molar ratio

The following benzene hydrogenation examples were conducted at different Brij 35 / TPPTS molar ratios. The reaction conditions were the following:
○ T = 130° C
○ P_{H2} = 80 bar
○ Hydrogenation time = 5 min
○ 2.63 mg (0.01 mmol) RhCl₃ 3H₂O
○ 24.89 mg (0.04 mmol) TPPTS (P/Rh molar ratio = 4)
○ 3 g deaerated distilled demineralized water
○ [Rh] = 340 ppm
○ Stirring rate = 670 rpm

Additional details of the hydrogenation and the results of the trials are compiled in Table 5:

**Table 5**

| Biphasic hydrogenation of benzene catalyzed by Rh/TPPTS complexes at different Brij35/TPPTS molar ratios | | | | | | |
|---|---|---|---|---|---|---|
| **Ex.** | **Catalyst Precursor** | **C=C/Rh molar ratio** | **Surfactant/TPPTS molar ratio** | **Conversion** **(mol %)** | **Selectivity of cyclohexane** **(mol %)** | **TOF^{a}** **(h⁻¹)** |
| 22 | RhCl₃·3H₂O/TPPTS | 30000 | Brij35^{b} /TPPTS =0.3 | 45 | 100 | 162330 |
| 23 | RhCl₃·3H₂O/TPPTS | 24000 | Brij35^{b} /TPPTS =1 | 65 | 100 | 186600 |
| 24 | RhCl₃·3H₂O/TPPTS | 24000 | Brij35^{b} /TPPTS =2 | 68 | 100 | 195390 |

| | | | | | | |
|---|---|---|---|---|---|---|
| ^{a} Defined as mole of hydrogenated C=C units of benzene per mole of Rh per hour. ^{b} Brij 35 = polyoxyethylene(23)lauryl ether. | | | | | | |

### Examples 25 - 26

### Selective hydrogenation of benzene to cyclohexane - Influence of Brij 30/TPPTS molar ratio

The following benzene hydrogenation examples were conducted at different Brij 30 / TPPTS molar ratios. The reaction conditions were the following:
○ T =130° C
○ P_{H2} = 80 bar
○ Hydrogenation time = 5 min
○ 2.63 mg (0.01 mmol) RhCl₃· 3H₂O
○ 24.89 mg (0.04 mmol) TPPTS (P/Rh molar ratio = 4)
○ 7.81 g (100 mmol) benzene (C=C/Rh molar ratio = 30000)
○ 3 g deaerated distilled demineralized water
○ [Rh] = 340 ppm
○ Stirring rate = 670 rpm

Additional details of the hydrogenation and the results of the trials are compiled in Table 6:

**Table 6**

| Biphasic hydrogenation of benzene catalyzed by Rh/TPPTS complexes at different Brij30/TPPTS molar ratios | | | | | | |
|---|---|---|---|---|---|---|
| **Ex.** | **Catalyst Precursor** | **C=C/Rh molar ratio** | **Surfactant/TPPTS molar ratio** | **Conversion** **(mol %)** | **Selectivity of cyclohexane** **(mol %)** | **TOF^{a}** **(h⁻¹)** |
| 25 | RhCl₃·3H₂O/TPPTS | 30000 | Brij30/TPPTS =1 | 55 | 100 | 197400 |
| 26 | RhCl₃·3H₂O/TPPTS | 30000 | Brij30/TPPTS =2 | 57 | 100 | 204400 |

| | | | | | | |
|---|---|---|---|---|---|---|
| ^{a} Defined as mole of hydrogenated C=C units of benzene per mole of Rh per hour. | | | | | | |

### Examples 27 - 30

### Selective hydrogenation of benzene to cyclohexane - Influence of the volume ratio of the aqueous to the organic phase

The following benzene hydrogenation examples were carried out under different volume ratios of the aqueous to the organic phase. The reaction conditions were the following:
○ 2.63 mg (0.01 mmol) RhCl₃·3H₂O
○ Catalyst precursor RhCl₃·3H₂O/TPPTS
○ Stirring rate = 670 rpm

Additional details of the hydrogenation and the results of the trials are compiled in Table 7:

**Table 7**

| Biphasic hydrogenation of benzene catalyzed by Rh/TPPTS at different volume ratios of the aqueous to the organic phase | | | | | | | | | | |
|---|---|---|---|---|---|---|---|---|---|---|
| **Ex.** | **P/Rh molar ratio** | **C=C/Rh molar ratio** | **V_{aq}/V_{org}** **(ml/ml)** | **T** **(°C)** | **P_{H2}** **(bar)** | **t** **(min)** | **H₂O** **(g)** | **Convers.** **(mol %)** | **Selectivity of cyclohexane** **(mol %)** | **TOF^{a}** **(h⁻¹)** |
| 27 | 8 | 15000 | 4.5 | 120 | 50 | 10 | 20 | 53 | 100 | 48180 |
| 28 | 8 | 15000 | 2.2 | 120 | 50 | 10 | 10 | 83 | 100 | 74790 |
| 29 | 8 | 15000 | 1.1 | 120 | 50 | 10 | 5 | 92 | 100 | 82470 |
| 30 | 4 | 30000 | 0.7 | 130 | 80 | 5 | 3 | 38 | 100 | 135690 |

| | | | | | | | | | | |
|---|---|---|---|---|---|---|---|---|---|---|
| ^{a} Defined as mole of hydrogenated C=C units of benzene per mole of Rh per hour. | | | | | | | | | | |

### Examples 31- 36

### Selective hydrogenation of benzene to cyclohexane - Influence of C=C/Rh molar ratio

The following benzene hydrogenation examples were carried out at different C=C/Rh molar ratios. The reaction conditions were the following:
○ 2.63 mg (0.01 mmol) RhCl₃·3H₂O
○ Catalyst precursor RhCl₃·3H₂O/TPPTS
○ Stirring rate = 670 rpm

Additional details of the hydrogenation and the results of the trials are compiled in Table 8:

**Table 8**

| Biphasic hydrogenation of benzene catalyzed by Rh/TPPTS at different C=C/Rh molar ratios | | | | | | | | | |
|---|---|---|---|---|---|---|---|---|---|
| **Ex.** | **P/Rh molar ratio** | **C=C/Rh molar ratio** | **T** **(°C)** | **P_{H2}** (bar) | **t** **(min)** | **H₂O** **(g)** | **Conversion** **(mol %)** | **Selectivity cyclohexane** **(mol %)** | **TOF^{a}** **(h⁻¹)** |
| 31 | 3 | 9000 | 130 | 30 | 10 | 10 | 98 | 100 | 53010 |
| 32 | 3 | 15000 | 120 | 80 | 10 | 10 | 96 | 100 | 86790 |
| 33 | 4 | 24000 | 130 | 80 | 5 | 3 | 48 | 100 | 136680 |
| 34 | 4 | 30000 | 130 | 80 | 5 | 3 | 38 | 100 | 135690 |
| 35 | 3 | 36000 | 130 | 80 | 5 | 5 | 30 | 100 | 128160 |
| 36 | 4 | 60000 | 130 | 80 | 5 | 5 | 5 | 100 | 38760 |

| | | | | | | | | | |
|---|---|---|---|---|---|---|---|---|---|
| ^{a} Defined as mole of hydrogenated C=C units of benzene per mole of Rh per hour | | | | | | | | | |

### Examples 37 - 41

### Selective hydrogenation of benzene to cyclohexane - Influence of the C=C/Rh molar ratio in the presence of Brij30 and Brij35

The following benzene hydrogenation examples were carried out at different C=C/Rh molar ratios in the presence of Brij 30 and Brij 35. The reaction conditions were the following:
○ T = 130° C
○ P_{H2} = 80 bar
○ Hydrogenation time = 5 min
○ 2.63 mg (0.01 mmol) RhCl₃· 3H₂O
○ 24.89mg (0.04 mmol) TPPTS (P/Rh molar ratio = 4)
○ 3 g deaerated distilled demineralized water
○ [Rh] = 340 ppm; stirring rate = 670 rpm

Additional details of the hydrogenation and the results of the trials are compiled in Table 9:

**Table 9**

| Biphasic hydrogenation of benzene catalyzed by Rh/TPPTS complexes at different C=C/Rh molar ratios in the presence of Brij30 and Brij35 | | | | | | |
|---|---|---|---|---|---|---|
| **Ex.** | **Catalyst Precursor** | **C=C/Rh molar ratio** | **Surfactant/ TPPTS molar ratio** | **Conversion** **(mol %)** | **Selectivity cyclohexane** **(mol %)** | **TOF^{a}** **(h⁻¹)** |
| 37 | RhCl₃·3H₂O/TPPTS | 24000 | Brij35/TPPTS =2 | 68 | 100 | 195390 |
| 38 | RhCl₃·3H₂O/TPPTS | 30000 | Brij35/TPPTS =2 | 52 | 100 | 187530 |
| 39 | RhCl₃·3H₂O/TPPTS | 27000 | Brij30/TPPTS =2 | 60 | 100 | 193830 |
| 40 | RhCl₃·3H₂O/TPPTS | 30000 | Brij30/TPPTS =2 | 57 | 100 | 204400 |
| 41 | RhCl₃·3H₂O/TPPTS | 33000 | Brij30/TPPTS =2 | 49 | 100 | 195090 |

| | | | | | | |
|---|---|---|---|---|---|---|
| ^{a} Defined as mole of hydrogenated C=C units of benzene per mole of Rh per hour. | | | | | | |

### Examples 42-44

### Selective hydrogenation of benzene to cyclohexane - Influence of the pH value

The following benzene hydrogenation examples were carried out at different pH values of the aqueous catalyst solution. The reaction conditions were the following:
○ T = 120°C; P_{H2}= 50 bar; Hydrogenation time = 10 min
○ 2.63mg (0.01 mmol) RhCl₃-3H₂O; 49.8mg (0.08 mmol) TPPTS (P/Rh molar ratio = 8)
○ 6.25 g (80 mmol) of benzene (C=CRh molar ratio = 24000)
○ 10 g deaerated distilled demineralized water; [Rh] =100 ppm; Stirring rate = 670 rpm

Additional details of the hydrogenation and the results of the trials are compiled in Table 10:

**Table 10**

| Effect of the pH on the hydrogenation of benzene catalyzed by water soluble Rh/TPPTS catalytic complexes in aqueous/organic two-phase systems | | | | | | |
|---|---|---|---|---|---|---|
| **Ex.** | **Catalyst Precursor** | **C=C/Rh molar ratio** | **pH value** | **Conversion** **(mol %)** | **Selectivity cyclohexane** **(mol %)** | **TOF²** **(h⁻¹)** |
| 42 | RhCl₃-3H₂O/TPPTS | 24000 | 3.5^{b} | 88 | 100 | 52920 |
| 43 | RhCl₃·H₂O/TPPTS | 24000 | 7.0^{c} | 87 | 100 | 42390 |
| 44 | RhCl₃·3H₂O/TPPTS | 24000 | 12.0^{d} | 83 | 100 | 39480 |

| | | | | | | |
|---|---|---|---|---|---|---|
| ^{a} Defined as mole of hydrogenated C=C units per mole of Rh per hour. ^{b} No buffer was added. ^{c} 110.4 mg (0.8 mmol) NaH₂PO₄·H₂O, pH adjusted with 5% aqueous NaOH; Rhodium black was observed. ^{d} Adjusted with 5% aqueous NaOH; Formation of massive rhodium black observed, indicating of heterogeneous catalytic particles. | | | | | | |

The effect that at increasing pH values the TOF decreases (examples 42 - 44) suggests a homogeneous nature for the Rh/TPPTS catalyst in aqueous media at acidic conditions. This pH effect is used as an evaluation method to decide for the presence of a homogeneous catalyst or for nanoparticles. C. Daguenet and P.J. Dyson [Catal. Commun. 4, pl53f (2003)] developed this pH evaluation method as a screen to help to assess for the homogeneous nature of transition metal catalytic complexes versus heterogeneous nanoparticles. Moreover, at pH = 7.0 and 12.0 (examples 43 and 44) rhodium black was observed after the reaction indicating for the presence of heterogeneous catalysts at neutral and basic conditions.

### Examples 45 - 46

### Selective hydrogenation of benzene to cyclohexane - Recycling of the Rh/TPPTS catalyst

The Rh/TPPTS catalyst after the benzene hydrogenation reaction could be easily recovered by a simple phase separation and a recycling experiment of the aqueous Rh/TPPTS catalyst solution showed that the catalytic activity remained high in a consecutive run. The reaction conditions were the following:
○ T =120° C
○ P_{H2}= 60 bar
○ Hydrogenation time =10 min
○ 2.63 mg (0.01 mmol) RhCl₃·3H₂O;
○ 24.89 mg (0.04 mmol) TPPTS (P/Rh molar ratio = 4)
○ 3.91 g (50 mmol) benzene (C=C/Rh molar ratio =15000)
○ 10 g deaerated distilled demineralized water
○ [Rh] =100 ppm; Stirring rate = 670 rpm

Additional details of the hydrogenation and the results of the trials are compiled in Table 11:

**Table 11**

| Recycling of aqueous Rh/TPPTS catalyst solution in the hydrogenation of benzene in aqueous/organic two phase systems | | | | | |
|---|---|---|---|---|---|
| **Ex.** | **Catalyst Precursor** | **C=C/Rh molar ratio** | **Conversion** **(mol %)** | **Selectivity cyclohexane** **(mol %)** | **TOF^{a}** **(h⁻¹)** |
| 45 | RhCl₃·3H₂O/TPPTS | 15000 | 83 | 100 | 75060 |
| 46 | Recycled catalyst | 15000 | 84 | 100 | 75570 |

| | | | | | |
|---|---|---|---|---|---|
| ^{a} Defined as mole of hydrogenated C=C units per mole of Rh per hour. | | | | | |

### Example 47

### Dynamic light scattering studies on a five times recycled aqueous Rh/TPPTS catalyst solution

Dynamic light scattering studies were carried out on the aqueous Rh/TPPTS catalyst solution which was recycled five times. The Rh/TPPTS catalyst after each benzene hydrogenation reaction could be easily recovered by a simple phase separation and the five recycling experiments of the aqueous catalyst layer showed that the catalytic activity remained high in all five consecutive runs. The reaction conditions were the following:
○ T =120 °C
○ P_{H2} = 60 bar
○ Hydrogenation time = 10 min
○ 2.63 mg (0.01 mmol) RhCl₃·3H₂O;
○ 49.3 mg (0.08 mmol) TPPTS (TPPTS/Rh molar ratio = 8)
○ 10 g deaerated distilled demineralized water
○ pH of the aqueous catalyst solution = 3.5
○ 3.91 g (50 mmol) benzene (C=C/Rh molar ratio = 15000)
○ [Rh] =100 ppm; Stirring rate = 670 rpm

Dynamic light scattering studies on the aqueous Rh/TPPTS catalyst solution which was recycled five times after six consecutive biphasic hydrogenation reactions of benzene suggested that the Rh/TPPTS catalyst is of a homogeneous nature in aqueous media without formation of any heterogeneous rhodium catalytic nanoparticles.

Measurements were conducted with a Series 4700 Malvern system composed of a PCS5101 goniometer with a PCS stepper motor controller, a Cyonics variable power Ar⁺ laser, operating at 488 nm, a PCS8 temperature control unit, a RR98 pump/filtering unit and a 192 channel correlator for the accumulation of the data. The correlation functions were analyzed by the cumulant method and CONTIN software. Measurements were carried out at 45°, 90° and 135°.

### Examples 48 - 54

### Selective hydrogenation of methyl benzoate to methyl cyclohexanoate - Influence of hydrogen partial pressure

The following methyl benzoate hydrogenation examples were carried out under various hydrogen partial pressures. The reaction conditions were the following:
○ T =120 °C
○ Hydrogenation time =10 min
○ 2.63mg (0.01 mmol) RhCl₃·3H₂O; 18.5mg (0.03 mmol) TPPTS (P/Rh molar ratio= 3)
○ 1.36 g (10 mmol) methyl benzoate
○ 10 g deaerated distilled demineralized water
○ 3.44 mg (0.02 mmol) *p*-toluenesulphonic acid
○ [Rh] = 100 ppm; stirring rate = 670 rpm

Additional details of the hydrogenation and the results of the trials are compiled in Table 12:

**Table 12**

| Biphasic hydrogenation of methyl benzoate catalyzed by Rh/TPPTS under different H₂ pressure | | | | | | |
|---|---|---|---|---|---|---|
| **Ex.** | **Catalyst Precursor** | **C=C/Rh molar ratio** | **P_{H2}** **(bar)** | **Conversion** **(mol %)** | **Selectivity of methyl cyclohexanoate** **(mol %)** | **TOF^{a}** **(h⁻¹)** |
| 48 | RhCl₃·3H₂O/TPPTS | 3000 | 10 | 11 | 100 | 1950 |
| 49 | RhCl₃·₃H₂O/TPPTS | 3000 | 30 | 36 | 100 | 6450 |
| 50 | RhCl₃·3H₂O/TPPTS | 3000 | 50 | 52 | 100 | 9120 |
| 51 | RhCl₃·3H₂O/TPPTS | 3000 | 60 | 51 | 100 | 9450 |
| 52 | RhCl₃·3H₂O/TPPTS | 3000 | 70 | 55 | 100 | 9960 |
| 53 | RhCl₃·3H₂O/TPPTS | 3000 | 80 | 62 | 100 | 11160 |
| 54 | RhCl₃·3H₂O/TPPTS | 3000 | 90 | 64 | 100 | 11460 |

| | | | | | | |
|---|---|---|---|---|---|---|
| ^{a} Defined as mole of hydrogenated C=C units of methyl benzoate per mole of Rh per hour. | | | | | | |

### Examples 55 - 60

### Selective hydrogenation of methyl benzoate to methyl cyclohexanoate - Influence of temperature

The following methyl benzoate hydrogenation examples were carried out under various temperatures. The reaction conditions were the following:
○ P_{H2}= 30 bar
○ Hydrogenation time =10 min
○ 2.63mg (0.01 mmol) RhCl₃·3H₂O; 18.5mg (0.03 mmol) TPPTS (P/Rh molar ratio= 3)
○ 1.36 g (10 mmol) methyl benzoate
○ 10 g deaerated distilled demineralized water
○ 3.44 mg (0.02 mmol) *p*-toluenesulphonic acid
○ [Rh] = 100 ppm; stirring rate = 670 rpm

Additional details of the hydrogenation and the results of the trials are compiled in Table 13:

**Table 13**

| Biphasic hydrogenation of methyl benzoate catalyzed by Rh/TPPTS at different temperatures | | | | | | |
|---|---|---|---|---|---|---|
| **Ex.** | **Catalyst Precursor** | **C=C/Rh molar ratio** | **T(°C)** | **Conversion** **(mol %)** | **Selectivity of methyl cyclohexanoate** **(mol %)** | **TOF ^{a}** **(h⁻¹)** |
| 55 | RhCl₃·3H₂O/TPPTS | 3000 | 80 | 18 | 100 | 3210 |
| 56 | RhCl₃·3H₂O/TPPTS | 3000 | 90 | 22 | 100 | 3960 |
| 57 | RhCl₃·3H₂O/TPPTS | 3000 | 100 | 24 | 100 | 4350 |
| 58 | RhCl₃·3H₂O/TPPTS | 3000 | 110 | 24 | 100 | 4380 |
| 59 | RhCl₃·3H₂O/TPPTS | 3000 | 120 | 36 | 100 | 6450 |
| 60 | RhCl₃·3H₂O/TPPTS | 3000 | 130 | 34 | 100 | 6060 |

| | | | | | | |
|---|---|---|---|---|---|---|
| ¹ Defined as mole of hydrogenated C=C units of methyl benzoate per mole of Rh per hour. | | | | | | |

### Examples 61 - 66

### Selective hydrogenation of methyl benzoate to methyl cyclohexanoate - Influence of reaction time

The following methyl benzoate hydrogenation examples were carried out under various reaction times. The reaction conditions were the following:
○ T =100°C
○ P_{H2}=30 bar
○ 2.63mg (0.01 mmol) RhCl₃·3H₂O, 18.5mg (0.03 mmol) TPPTS (P/Rh molar ratio= 3)
○ 10 g deaerated distilled demineralized water
○ [Rh] =100 ppm; stirring rate = 670 rpm

Additional details of the hydrogenation and the results of the trials are compiled in Table 14:

**Table 14**

| Biphasic hydrogenation of methyl benzoate catalyzed by Rh/TPPTS at different reaction times | | | | | | | |
|---|---|---|---|---|---|---|---|
| **Ex.** | **Catalyst Precursor** | **C=C/Rh molar ratio** | ***p*-toluene sulphonic acid** **(mmol)** | **t (min)** | **Conversion** **(mol %)** | **Selectivity of methyl cyclohexanoate** **(mol %)** | **TOF^{a}** **(h⁻¹)** |
| 61 | RhCl₃·3H₂O/TPPTS | 1000 | 0.02 | 10 | 24 | 100 | 4350 |
| 62 | RhCl₃·3H₂O/TPPTS | 1000 | 0.02 | 20 | 33 | 100 | 3030 |
| 63 | RhCl₃·3H₂O/TPPTS | 1000 | 0.02 | 40 | 54 | 100 | 2460 |
| 64 | RhCl₃·3H₂O/TPPTS | 1000 | 0.02 | 60 | 67 | 100 | 2010 |
| 65 | RhCl₃·3H₂O/TPPTS | 500 | - | 20 | 44 | 100 | 1980 |
| 66 | RhCl₃·3H₂O/TPPTS | 500 | - | 60 | 83 | 100 | 1260 |

| | | | | | | | |
|---|---|---|---|---|---|---|---|
| ^{a} Defined as mole of hydrogenated C=C units of methyl benzoate per mole of Rh per hour. | | | | | | | |

### Examples 67 - 70

### Selective hydrogenation of methyl benzoate to methyl cyclohexanoate - Influence of TPPTS/Rh molar ratio

The following methyl benzoate hydrogenation examples were carried out under various TPPTS/Rh molar ratios. The reaction conditions were the following:
○ T=100°C
○ P_{H2}= 30 bar
○ Hydrogenation time =10 min
○ 2.63 mg (0.01 mmol) RhCl₃·3H₂O
○ 1.36 g (10 mmol) methyl benzoate
○ 10 g deaerated distilled demineralized water
○ 3.44 mg (0.02 mmol) p-toluenesulphonic acid
○ [Rh] =100 ppm; stirring rate = 670 rpm

Additional details of the hydrogenation and the results of the trials are compiled in Table 15:

**Table 15**

| Biphasic hydrogenation of methyl benzoate catalyzed by Rh/TPPTS at various P/Rh molar ratios | | | | | | |
|---|---|---|---|---|---|---|
| **Ex.** | **Catalyst Precursor** | **TPPTS/Rh molar ratio** | **C=C/Rh molar ratio** | **Conversion** **(mol %)** | **Selectivity of methyl cyclohexanoate** **(mol %)** | **TOF^{a}** |
| 67 | RhCl₃·3H₂O/TPPTS | 3 | 3000 | 24 | 100 | 4350 |
| 68 | RhCl₃·3H₂O/TPPTS | 4 | 3000 | 17 | 100 | 3090 |
| 69 | RhCl₃·3H₂O/TPPTS | 5 | 3000 | 15 | 100 | 2790 |
| 70 | RhCl₃·3H₂O.TPPTS | 8 | 3000 | 20 | 100 | 3660 |

| | | | | | | |
|---|---|---|---|---|---|---|
| ^{a} Defined as mole of hydrogenated C=C units of methyl benzoate per mole of Rh per hour. | | | | | | |

### Examples 71 - 76

### Selective hydrogenation of methyl benzoate to methyl cyclohexanoate - Recycling of the Rh/TPPTS catalyst

The Rh/TPPTS catalyst after the methyl benzoate hydrogenation reaction could be easily recovered by a simple phase separation and five recycling experiments of the aqueous Rh/TPPTS catalyst solution showed that the catalytic activity remained high all five consecutive run. The reaction conditions were the following:
○ T = 120° C
○ P_{H2} = 75 bar
○ Hydrogenation time = 10 min
○ 2.63mg (0.01 mmol) RhCl₃·3H₂O; 24.6mg (0.04 mmol) TPPTS (P/Rh molar ratio= 4)
○ 1.36 g (10 mmol) methyl benzoate
○ 10 g deaerated distilled demineralized water
○ 3.44 mg (0.02 mmol) p-toluenesulphonic acid
○ [Rh] = 100 ppm; stirring rate = 670 rpm

Additional details of catalyst recycling and the results of the trials are compiled in Table 16:

**Table 16**

| Recycling of the aqueous Rh/TPPTS catalyst solution in the hydrogenation of methyl benzoate in aqueous/organic two phase systems | | | | | |
|---|---|---|---|---|---|
| **Ex.** | **Catalyst Precursor** | **C=C/Rh molar ratio** | **Conversion** **(mol %)** | **Selectivity of methyl cyclohexanoate** **(mol %)** | **TOF^{a}** |
| 71 | RhCl₃·3H₂O/TPPTS | 3000 | 55 | 100 | 9840 |
| 72 | Recycled catalyst | 3000 | 51 | 100 | 9150 |
| 73 | Recycled catalyst | 3000 | 47 | 100 | 8400 |
| 74 | Recycled catalyst | 3000 | 52 | 100 | 9390 |
| 75 | Recycled catalyst | 3000 | 49 | 100 | 8910 |
| 76 | Recycled catalyst | 3000 | 52 | 100 | 9330 |

| | | | | | |
|---|---|---|---|---|---|
| ^{a} Defined as mole of hydrogenated C=C units of methyl benzoate per mole of Rh per hour. | | | | | |

### Examples 77 - 80

### Selective hydrogenation of methyl benzoate to methyl cyclohexanoate -Influence of the pH value

The following methyl benzoate hydrogenation examples were carried out at different pH values of the aqueous catalyst solution. The reaction conditions were the following:
○ T=100°C; P_{H2}= 30 bar; Hydrogenation time = 20 min
○ 2.63mg (0.01 mmol) RhCl₃·3H₂O; 18.5mg (0.03 mmol) TPPTS (P/Rh molar ratio= 3)
○ 0.68 g (5 mmol) methyl benzoate
○ 10 g deaerated distilled demineralized water
○ [Rh] =100 ppm; stirring rate = 670 rpm

Additional details of the hydrogenation and the results of the trials are compiled in Table 17:

**Table 17**

| Biphasic hydrogenation of methyl benzoate catalyzed by Rh/TPPTS at different pH values | | | | | | |
|---|---|---|---|---|---|---|
| **Ex.** | **Catalyst Precursor** | **C=C/Rh molar ratio** | **pH value** | **Conversion** **(mol %)** | **Selectivity methyl cyclohexanoate** **(mol %)** | **TOF^{a}** **(h⁻¹)** |
| 77 | RhCl₃·3H₂O/TPPTS | 1500 | 5.9^{b} | 53 | 100 | 2370 |
| 78 | RhCl₃·3H₂O/TPPTS | 1500 | 3.3 | 44 | 100 | 1970 |
| 79 | RhCl₃·3H₂O/TPPTS | 1500 | 2.9^{c} | 68 | 100 | 3060 |
| 80 | RhCl₃·3H₂O/TPPTS | 1500 | 2.5^{d} | 88 | 100 | 3960 |

| | | | | | | |
|---|---|---|---|---|---|---|
| ^{a} Defined as mole of hydrogenated C=C units of methyl benzoate per mole of Rh per hour. ^{b} 13.8 mg (0.1 mmol) NaH₂PO₄·H₂O. ^{c} 3.44 mg (0.02 mmol) *p*-toluenesulphonic acid. ^{d} 6.89 mg (0.04 mmol) of *p*-toluenesulphonic acid | | | | | | |

The effect that at increasing pH values the TOF decreases (examples 78 - 80) suggests a homogeneous nature for the Rh/TPPTS catalyst in aqueous media at acidic conditions. At pH > 7.0 saponification side reactions were observed during the hydrogenation reaction to give after acidification and the working up procedure as products benzoic acid, cyclohexanecarboxylic acid and methyl cyclohexanoate. Furthermore, at pH > 7.0 rhodium black was observed after the hydrogenation reaction indicating for the presence of heterogeneous catalysts at neutral and basic conditions.

### Examples 81-110

### Micellar hydrogenation of dimer fatty acid methyl esters catalyzed by various catalysts in aqueous/organic two phase systems

The following dimer fatty acid methyl ester hydrogenation examples were carried out in the presence of various catalysts in aqueous/organic two phase systems. The reaction conditions were the following:
○ 13.2mg (0.05 mmol) RhCl₃·3H₂O; 13.05mg (0.05 mmol) RuCl₃·3H₂O; 19.9mg (0.05 mmol) Ru(acac)₃; 17.6mg (0.05mmol) IrCk₃·3H₂O; 11.2mg (0.05 mmol) Pd(OAc)₂; 12.4mg (0.05 mmol )Ni(OAc)₂; 12.45mg (0.05 mmol) Co(OAc)₂; 8.69mg (0.05 mmol) Fe(OAc)₂ ; 17.89 mg (0.05 mmol) HAuCl₄·H₂O; 13.3 mg (0.05 mmol) PtCl₂;
○ 53.2 mg of commercial 10 wt. % Pd/C
○ 22.4 mg (0.07 mmol) CTAC; 8.40 mg (0.007 mmol) Brij 35; 5.6 mg (0.005 mmol) Brij 58; 5.75 mg (0.005 mmol) Brij 78; 2.17 g (6 mmol) Brij 30
○ 1 g dimer fatty acid methyl ester; except entry 110 with 0.5 g substrate
○ 50 g deaerated distilled demineralized water
○ [Pt] =190 ppm; Stirring rate = 670 rpm

Additional details of the hydrogenation and the results of the trials are compiled in Table 18.

***Product analysis:*** ¹H NMR (300 MHz) were recordered on a Varian Unity Plus 300/54 spectrometer. The conversions in the hydrogenation of dimer fatty acid methyl ester were determined by quantitative ¹H NMR analysis after addition of ethylene carbonate internal standard in CDCl₃; 4.0 - 4.4 ppm and 5.0 - 5.5 ppm: olefinic protons from both cyclic and acyclic dimer fatty acid methyl esters; 4.5 ppm: protons of the internal standard ethylene carbonate; 6.6 - 7.2 ppm: aromatic protons of the dimer fatty acid methyl esters aromatic compounds.

**Table 18**

| Micellar hydrogenation ofdimer fatty acid methyl esters (dimer) employing various catalysts in aqueous/organic two-phase systems | | | | | | | | | |
|---|---|---|---|---|---|---|---|---|---|
| **Ex.** | **Catalyst Precursor** | **P/Metal molar ratio** | **T** **(°C)** | **P_{H2}** **(bar)** | **t** **(min)** | **Surfactant** | **Unreacted aromatics** **(mol%)** | **Unreacted olefinics** **(mol%)** | **Conversion** **(mol%)** |
| Dimer | - | - | - | - | - | - | 42 | 58 | - |
| 81 | Pd/C | - | 100 | 80 | 60 | - | 11 | 5 | 84 |
| 82^{a} | Pd/C | - | 100 | 80 | 60 | - | 16 | 24 | 60 |
| 83 | RhCl₃·3H₂O/TPPTS | 4 | 100 | 80 | 60 | CTAC | 14 | 19 | 67 |
| 84 | RhCl₃·3H₂O/TPPTS | 4 | 100 | 80 | 60 | CTAC | 10 | 15 | 75 |
| 85 | RhCl₃·3H₂O/TPPTS | 4 | 130 | 80 | 60 | CTAC | 10 | 14 | 76 |
| 86 | RhCl₃·3H₂O/TPPTS | 4 | 80 | 80 | 60 | CTAC | 8 | 16 | 76 |
| 87 | RhCl₃·3H₂O/TPPTS | 4 | 90 | 80 | 60 | CTAC | 11 | 19 | 70 |
| 88 | RuCl₃·3H₂O/TPPTS | 4 | 80 | 80 | 60 | CTAC | 10 | 11 | 79 |
| 89 | RuCl₃·3H₂O/TPPTS | 4 | 90 | 80 | 60 - | CTAC | 11 | 20 | 69 |
| 90 | RuCl₃·3H₂O/TPPTS | 3 | 100 | 80 | 60 | CTAC | 6 | 12 | 82 |
| 91 | RuCl₃·3H₂O/TPPTS | 4 | 100 | 50 | 60 | CTAC | 19 | 26 | 55 |
| 92 | Ru(acac)₃/TPPTS | 4 | 100 | 80 | 60 | CTAC | 9 | 12 | 79 |
| 93^{b} | IrCl₃·3H₂O/TPPTS | 4 | 100 | 80 | 60 | CTAC | 19 | 25 | 56 |
| 94^{b} | Pd(OAc)₂/TPPTS | 4 | 100 | 80 | 60 | CTAC | 23 | 23 | 54 |
| 95^{b} | Ni(OAc)₂·4H₂O/TPPTS | 4 | 100 | 80 | 60 | CTAC | 21 | 30 | 49 |
| 96^{b} | Co(OAc)₂·4H₂O/TPPTS | 4 | 100 | 80 | 60 | CTAC | 6 | 9 | 85 |
| 97^{b} | Fe(OAc)₂/TPPTS | 4 | 100 | 80 | 60 | CTAC | 10 | 16 | 74 |
| 98^{b} | HAuCl₄·H₂O/TPPTS | 4 | 100 | 80 | 60 | CTAC | 26 | 36 | 38 |
| 99^{b} | PtCl₂/TPPTS | 4 | 100 | 80 | 60 | CTAC | 19 | 9 | 72 |
| 100 | PtCl₂/TPPTS | 4 | 80 | 80 | 60 | CTAC | 21 | 13 | 66 |
| 101 | PtCl₂/TPPTS | 4 | 120 | 80 | 60 | CTAC | 19 | 22 | 59 |
| 102 | PtCl₂/TPPTS | 4 | 80 | 40 | 60 | CTAC | 21 | 15 | 64 |
| 103 | PtCl₂/TPPTS | 4 | 70 | 80 | 60 | CTAC | 24 | 12 | 64 |
| 104 | PtCl₂/TPPTS | 5 | 80 | 80 | 120 | CTAC | 12 | 5 | 83 |
| 105 | PtCl₂/TPPTS | 4 | 80 | 80 | 60 | Brij 35 | 16 | 13 | 71 |
| 106 | PtCl₂/TPPTS | 4 | 100 | 80 | 60 | Brij 78 | 27 | 18 | 55 |
| 107 | PtCl₂/TPPTS | 4 | 100 | 80 | 90 | Brij 35 | 12 | 9 | 79 |
| 108 | PtCl₂/TPPTS | 4 | 100 | 80 | 60 | Brij 58 | 15 | 10 | 75 |

**Table 18**

| Micellar hydrogenation of dimer fatty acid methyl esters (dimer) employing various catalysts in aqueous/organic two-phase systems (Cont.) | | | | | | | | | |
|---|---|---|---|---|---|---|---|---|---|
| **Ex.** | **Catalyst Precursor** | **P/Metal molar ratio** | **T** **(°C)** | **P_{H2}** **(bar)** | **t** **(min)** | **Surfactant** | **Unreacted aromatics** **(mol%)** | **Unreacted olefinics** **(mol%)** | **Conversion** **(mol%)** |
| 109 | PtCl₂/TPPTS | 4 | 80 | 80 | 120 | CTAC | 6 | 3 | 91 |
| 110 | PtCl₂/TPPTS | 4 | 100 | 80 | 60 | Brij 35 | 2 | 6 | 92 |
| 111 | RhCl₃·3H₂O/TPPTS | 4 | 100 | 80 | 90 | Brij 30 | 0.7 | 0 | 99.3 |

| | | | | | | | | | |
|---|---|---|---|---|---|---|---|---|---|
| ^{a} 50 ml of THF was used as a solvent; several new unidentified products. ^{b} The results of the experiments as described in examples 93-99 are given without to take into account memory effects of the autoclave regarding the previously applied metal. | | | | | | | | | |

### Example 112

### Scale up of the micellar hydrogenation of dimer fatty acid methyl esters catalyzed by Ru/TPPTS in aqueous/organic two-phase systems

The micellar hydrogenation of dimer fatty acid methyl esters catalyzed by Ru/TPPTS in aqueous/organic two-phase systems was scaled up by a factor of 30, in an autoclave of a nominal volume of 2000 ml, according to the reaction as described in example 111 of table 18 except that RuCl₃·3H₂O was used at a TPPTS/Ru molar ratio of 3 under 65 bar hydrogen partial pressure, less amount of Brij 30 and 1400 ml of aqueous solvent. The reaction conditions were the following:
○ T =100° C
○ P_{H2} = 65 bar
○ Hydrogenation time = 90 min
○ 391.5 mg (1.5 mmol) RuCl₃·3H₂O;
○ 2.83 g (4.5 mmol) TPPTS (P/Ru molar ratio = 3)
○ 20 g (55.2 mmol) Brij 30
○ 15 g of dimer fatty acid methyl ester
○ 1400 g deaerated distilled demineralized water
○ [Ru] = 100 ppm
○ Stirring rate = 650 rpm

After the reaction and working up procedure by extraction with ether/hexane the conversion of dimer fatty acid methyl esters was 96 mol%, the unreacted aromatic compounds 0.95 mol% and the unreacted cyclic and acyclic olefins 3.05 mol%.

## Claims

1. Process for the manufacture of saturated mono- or polycyclic compounds via hydrogenation of the respective unsaturated mono- or polycyclic compounds, **characterised in that** the reaction is conducted in an aqueous/organic two phase system in the presence of a water soluble catalyst consisting of a Group VIII metal and a hydrophilic ligand.

2. Process according to Claim 1, **characterised in that** said monocyclic or polycyclic compounds follow at least one of the following general formulas or their geometric isomers 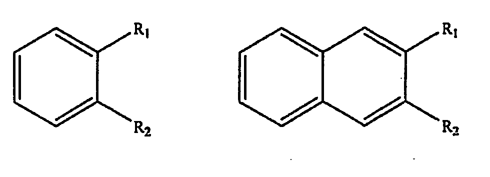 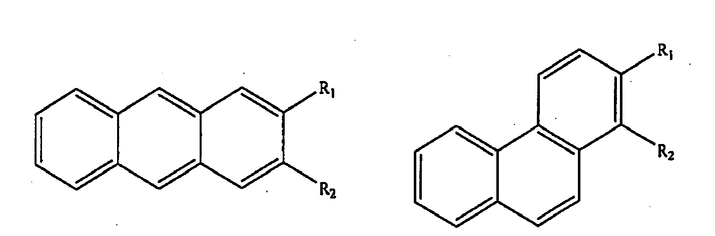 in which R¹ and R² independently from each other represent hydrogen or an alkyl, ester, alkoxy, aldehyde, keto, acid, amide, nitril, amino, nitro, halide, and/or hydroxy group.

3. Process according to Claim 1, **characterised in that** said monocyclic or polycyclic compounds represent dimer fatty acids or their esters.

4. Process according to any of the preceding Claims 1 to 3, **characterised in that** said Group VIII metal of said homogenous catalyst is selected from the group consisting of rhodium, ruthenium, palladium, cobalt, platinum, nickel, iridium, iron and their mixtures.

5. Process according to any of the preceding Claims 1 to 4, **characterised in that** said hydrophilic ligand of said homogenous catalyst is selected from the group consisting of hydrophilic phosphines, amines and oxygen containing ligands.

6. Process according to any of the preceding Claims 1 to 5, **characterised in that** said homogenous catalyst is a complex of rhodium or ruthenium or platinum and trisulphonated triphenylphosphine (TPPTS).

7. Process according to any of the preceding Claims 1 to 6, **characterised in that** the molar ratio between the Group VIII metal and the hydrophilic ligand is about 1:1 to about 1:16.

8. Process according to any of the preceding Claims 1 to 7, **characterised in that** the molar ratio of C=C/Group VIII metal is about 500 to about 60,000.

9. Process according to any of the preceding Claims 1 to 8, **characterised in that** the hydrogenation takes place in the presence of a co-catalyst, selected from the group consisting of cationic, zwitterionic, nonionic and anionic surfactants, ionic liquids or a phase transfer catalysts.

10. Process according to any of the preceding Claims 1 to 9, **characterised in that** said co-catalyst is a tetraalkylammonium salt or an esterquat.

11. Process according to any of the preceding Claims 1 to 10, **characterised in that** said cationic surfactant is octyltrimethylammonium chloride (OTAC), dodecyltrimethylammonium chloride (DTAC), tetradecyltrimethylammonium chloride, cetyltrimethylammonium chloride (CTAC), cetylpyridinium chloride, octadecyltrimethylammonium chloride, distearyldimethylammonium chloride, dicocoylmethylethoxymonium methosulphate or distearoylmethylethoxymonium methosulphate.

12. Process according to any of the preceding Claims 1 to 11, **characterised in that** the molar ratio between co-catalyst and ligand is 50:1 to 0.01:1.

13. Process according to any of the preceding Claims 1 to 12, **characterised in that** the hydrogenation takes place in an aqueous/organic two-phase system, formed by the aqueous catalyst solution and the mono- or polycyclic unsaturated compound.

14. Process according to any of the preceding Claims 1 to 13, **characterised in that** the ratio between the volume of the aqueous and the organic phase is 50:1 to about 0.5:1.

15. Process according to any of the preceding Claims 1 to 18, **characterised in that** once the hydrogenation has been finished the phases are separated, the organic phase comprising the hydrogenation product is purified and the aqueous phase comprising the catalyst is recycled.
